# EUROPEAN PATENT SPECIFICATION

(11) **EP 3 377 110 B1**
(45) Date of publication and mention of the grant of the patent: **06.01.2021**
(21) Application number: 16788099.6
(22) Date of filing: 27.10.2016
(51) Int. Cl.: A61K 9/51, A61K 47/26, A61K 47/28, A61K 9/14, A61K 31/58, C08F 120/50

(54) **NANOPARTICLES BASED ON OPTIONALLY ALKOXYLATED POLY(ALKYL CYANOACRYLATES) HAVING A DEFINED DEGREE OF POLYMERIZATION**
NANOPARTIKEL AUF DER BASIS VON OPTIONAL ALKOXYLIERTEN POLY(ALKYLCYANACRYLATEN) MIT DEFINIERTEM POLYMERISATIONSGRAD
NANOPARTICULES À BASE DE POLY(ALKYL CYANOACRYLATES) ÉVENTUELLEMENT ALCOXYLÉS DE DEGRÉ DE POLYMÉRISATION DÉFINI

(30) Priority: 20.11.2015 EP 15195601; 20.11.2015 US 201562258101 P
(43) Date of publication of application: 26.09.2018
(73) Proprietor: AbbVie Deutschland GmbH & Co. KG, 65189 Wiesbaden (DE)
(72) Inventor: CURIC, Anamarija, 67061 Ludwigshafen (DE); KELLER, Benjamin-Luca, 67061 Ludwigshafen (DE); MÜLLER, Thomas, 67061 Ludwigshafen (DE); ROSENBERG, Jörg, 67061 Ludwigshafen (DE)
(74) Representative: Reitstötter Kinzebach
(86) International application number: PCT/EP2016/075989
(87) International publication number: WO 2017/084854

(56) References cited:
- SAI LI ET AL: "In vitro release of protein from poly(butylcyanoacrylate) nanocapsules with an aqueous core", COLLOID AND POLYMER SCIENCE ; KOLLOID-ZEITSCHRIFT UND ZEITSCHRIFT FÜR POLYMERE, SPRINGER, BERLIN, DE, vol. 283, no. 5, 1 February 2005 (2005-02-01), pages 480-485, XP019340390, ISSN: 1435-1536, DOI: 10.1007/S00396-004-1173-5
- REIMOLD I ET AL: "Delivery of nanoparticles to the brain detected by fluorescence microscopy", EUROPEAN JOURNAL OF PHARMACEUTICS AND BIOPHARMACEUTICS, ELSEVIER SCIENCE PUBLISHERS B.V., AMSTERDAM, NL, vol. 70, no. 2, 1 October 2008 (2008-10-01), pages 627-632, XP025470910, ISSN: 0939-6411, DOI: 10.1016/J.EJPB.2008.05.007 [retrieved on 2008-06-05] cited in the application
- ANAMARIJA CURIC ET AL: "Development and lyophilization of itraconazole loaded poly(butylcyanoacrylate) nanospheres as a drug delivery system", EUROPEAN JOURNAL OF PHARMACEUTICAL SCIENCES., vol. 78, 11 July 2015 (2015-07-11), pages 121-131, XP55332230, NL ISSN: 0928-0987, DOI: 10.1016/j.ejps.2015.07.010
- Bernard Rvan ET AL: "Novel sub-ceiling temperature rapid depolymerization- repolymerization reactions of cyanoacrylate polymers", , 2 April 1996 (1996-04-02), pages 217-227, XP55332433, Retrieved from the Internet: URL:http://onlinelibrary.wiley.com/doi/10. 1002/marc.1996.030170404/full [retrieved on 2017-01-05]
- DOUGLAS R. ROBELLO ET AL: "Degradation and stabilization of polycyanoacrylates", JOURNAL OF POLYMER SCIENCE PART A: POLYMER CHEMISTRY, vol. 37, no. 24, 15 December 1999 (1999-12-15), pages 4570-4581, XP55332427, US ISSN: 0887-624X, DOI: 10.1002/(SICI)1099-0518(19991215)37:24<457 0::AID-POLA14>3.0.CO;2-#
- ANNE-MAGALI LAYRE ET AL: "Busulfan loading into poly(alkyl cyanoacrylate) nanoparticles: Physico-chemistry and molecular modeling", JOURNAL OF BIOMEDICAL MATERIALS RESEARCH. PART B: APPLIED BIOMATERIALS, vol. 79B, no. 2, 1 November 2006 (2006-11-01), pages 254-262, XP55332078, US ISSN: 1552-4973, DOI: 10.1002/jbm.b.30536 cited in the application

## Description

The present invention relates to nanoparticles based on optionally alkoxylated poly(alkyl cyanoacrylates) having a defined degree of polymerization and dispersity, a method for preparing such nanoparticles, compositions comprising such nanoparticles and medical uses thereof. The invention further relates to a method for preparing such polymers.

### BACKGROUND OF THE INVENTION

Nanoparticles have been studied as drug delivery systems and in particular as possible sustained release systems for targeting drugs to specific sites of action within the patient. The term "nanoparticles" is generally used to designate polymer-based particles having a diameter in the nanometer range. Nanoparticles include particles of different structure, such as nanospheres and nanocapsules. Nanoparticles based on biocompatible and biodegradable polymers such as poly(alkyl cyanoacrylates) have been studied over the past three decades and are of particular interest for biomedical applications (cf. Couvreur et al., J Pharm Pharmacol 31:331-332, 1979; Vauthier et al., Adv Drug Deliv Rev. 55:519-548, 2003). They can be prepared by nanoprecipitation of preformed poly(alkyl cyanoacrylate) or by miniemulsion polymerization (cf., e.g., Layre et al., J Biomed Mater Res 2006, Part B: Appl Biomater 79B:254-262; Reimold et al., Eur J Pharm Biopharm 70:627-632, 2008; Vauthier et al., Adv Drug Deliv Rev 55:519-548, 2003; Hansali et al., Colloids and Surfaces B: Biointerfaces 88:332-338, 2011). Li et al. (Colloid and Polymer Science 2005, 283 (5):480-485) describes a study examining factors which influence the *in vitro* release of bovine serum albumin (BSA) from BSA-Ioaded poly(butyl cyanoacrylate) nanocapsules.

Properties such as high drug-loading capacity, low cytotoxicity, chemical stability during formulation as well as a good controllability and reproducibility of their composition are crucial for nanoparticles intended for biomedical applications. It is therefore desirable to provide nanoparticle preparations having such advantageous properties.

### SUMMARY OF THE INVENTION

The inventors found that nanoparticles according to the claims which are prepared from optionally alkoxylated poly(alkyl cyanoacrylates) having a particular degree of polymerization (and thus a particular molecular mass) and a particular dispersity have especially advantageous properties with regard to, e.g., particle size, drug-loading capacity and cytotoxicity. The degree of polymerization also influences nanoparticle concentration and the chemical stability of the polymer matrix. Specifically, the inventors found that optionally alkoxylated poly(alkyl cyanoacrylates) comprising 330 monomeric constituents or less are chemically stable during nanoparticle formulation, while larger optionally alkoxylated poly(alkyl cyanoacrylates) are prone to depolymerization processes (at least when not capped at the C-H acidic end; see below). The inventors further found that optionally alkoxylated poly(alkyl cyanoacrylates) comprising 60 monomeric constituents or more can yield nanoparticles of a higher particle size, higher drug load and lower toxicity than smaller optionally alkoxylated poly(alkyl cyanoacrylates).

Accordingly, the invention provides a nanoparticle comprising:
(1) one or more than one polymer
   - comprising from 60 to 330 monomeric constituents selected from C₁-C₁₀-alkyl cyanoacrylates and C₁-C₆-alkoxy-C₁-C₁₀-alkyl cyanoacrylates, and
   - having a dispersity (Ð_{M}) of less than 3.0, and
(2) one or more than one cargo compound selected from pharmaceutically active agents, cosmetically active agents and nutritional supplements.

The inventors further found that nanoparticles, which are prepared from optionally alkoxylated poly(alkyl cyanoacrylates) having a particular degree of polymerization and whose C-H acidic ends are functionalized (capped), are even less cytotoxic and virtually stable against depolymerization processes.

Accordingly, the invention further provides a nanoparticle based on the polymer(s) (1), as defined above, where the C-H acidic end(s) of the polymer(s) (1) is/are functionalized with a linear or branched C₁-C₁₀-alkyl radical or a linear or branched C₂-C₁₀-alkenyl radical comprising at least one double bond of which at least one is terminal, where the C₁-C₁₀-alkyl radical and the C₂-C₁₀-alkenyl radical independently of each other are unsubstituted or substituted by one or more substituent(s) selected from C₁-C₁₀-alkoxy, C₁-C₁₀-alkylcarbonyl, C₁-C₁₀-alkoxycarbonyl and nitro.

The invention also provides a pharmaceutical composition comprising a plurality of nanoparticles of the present invention, and a pharmaceutically acceptable carrier.

The invention also provides the nanoparticle or the pharmaceutical composition of the present invention for use in therapy and/or prophylaxis, in particular for use in a method of treating an inflammatory bowel disease.

The inventors developed a method for preparing an optionally alkoxylated poly(alkyl cyanoacrylate) having a defined degree of polymerization. The method involves an anionic polymerization process in an aprotic water-miscible organic solvent or solvent mixture and provides very high yields. For example, preparation of poly(n-butyl 2-cyanoacrylate) (PBCA) according to this method typically yields about 90% PBCA having a well-defined degree of polymerization with good repeatability and reproducibility. In contrast, when preparing PBCA in water the degree of polymerization is virtually impossible to control and yields of PBCA with the desired properties are very low (about 20%).

Accordingly, the invention also provides a method for preparing a polymer having a defined degree of polymerization, the method comprising:
(i) providing a liquid phase monomer selected from C₁-C₁₀-alkyl cyanoacrylates and C₁-C₆-alkoxy-C₁-C₁₀-alkyl cyanoacrylates dissolved in an aprotic water-miscible organic solvent or a mixture of two or more aprotic water-miscible organic solvents, where the liquid phase may moreover contain one or more than one C₁-C₃-alkanol;
(ii) adding one or more than one base selected from metal hydroxides and metal alkoxides to the liquid phase, where, in case that the base is selected from metal hydroxides and the liquid phase provided in (i) does not contain any C₁-C₃-alkanols, the metal hydroxide(s) is/are added in the presence of one or more than one C₁-C₃-alkanol;
(iii) allowing an equilibrium of depolymerization and repolymerization processes to establish such that the polymer having a defined degree of polymerization is formed; and
(iv) quenching the polymerization reaction.

The invention also provides a method for preparing an optionally alkoxylated poly(alkyl cyanoacrylate) having a defined degree of polymerization, whose C-H acidic ends are functionalized (capped), the method comprising the steps as defined above, wherein the liquid phase obtained in step (iii) containing the polymer having a defined degree of polymerization is reacted with a terminator compound of the general formula X-R³, wherein X is a leaving group and R³ is a linear or branched C₁-C₁₀-alkyl radical or a linear or branched C₂-C₁₀-alkenyl radical comprising at least one double bond of which at least one is terminal, where the C₁-C₁₀-alkyl radical and the C₂-C₁₀-alkenyl radical independently of each other are unsubstituted or substituted by one or more substituent(s) selected from C₁-C₁₀-alkoxy, C₁-C₁₀-alkylcarbonyl, C₁-C₁₀-alkoxycarbonyl and nitro, prior to the quenching in step (iv).

The invention further provides a method for preparing nanoparticles, the method comprising:
(a) providing a hydrophobic liquid phase comprising:
   (1) one or more than one polymer:
      - comprising from 60 to 330 monomeric constituents selected from C₁-C₁₀-alkyl cyanoacrylates and C₁-C₆-alkoxy-C₁-C₁₀-alkyl cyanoacrylates, and
      - having a dispersity (Ð_{M}) of less than 3.0, and
   (2) one or more than one cargo compound selected from pharmaceutically active agents, cosmetically active agents and nutritional supplements
      dissolved in a water-immiscible organic solvent or a mixture of two or more water-immiscible organic solvents;
(b) providing a hydrophilic liquid phase;
(c) finely dispersing the hydrophobic liquid phase in the hydrophilic liquid phase so as to form an emulsion; and
(d) removing at least part of the organic solvent(s) from the emulsion so as to obtain a suspension of nanoparticles in the hydrophilic solvent.

The invention further provides a method for preparing nanoparticles based on capped polymers, the method comprising the steps as defined above, wherein the C-H acidic end(s) of the polymer(s) in the hydrophobic liquid phase of step (a) is/are functionalized with a linear or branched C₁-C₁₀-alkyl radical or a linear or branched C₂-C₁₀-alkenyl radical comprising at least one double bond of which at least one is terminal, where the C₁-C₁₀-alkyl radical and the C₂-C₁₀-alkenyl radical independently of each other are unsubstituted or substituted by one or more substituent(s) selected from C₁-C₁₀-alkoxy, C₁-C₁₀-alkylcarbonyl, C₁-C₁₀-alkoxycarbonyl and nitro.

### BRIEF DESCRIPTION OF THE FIGURES

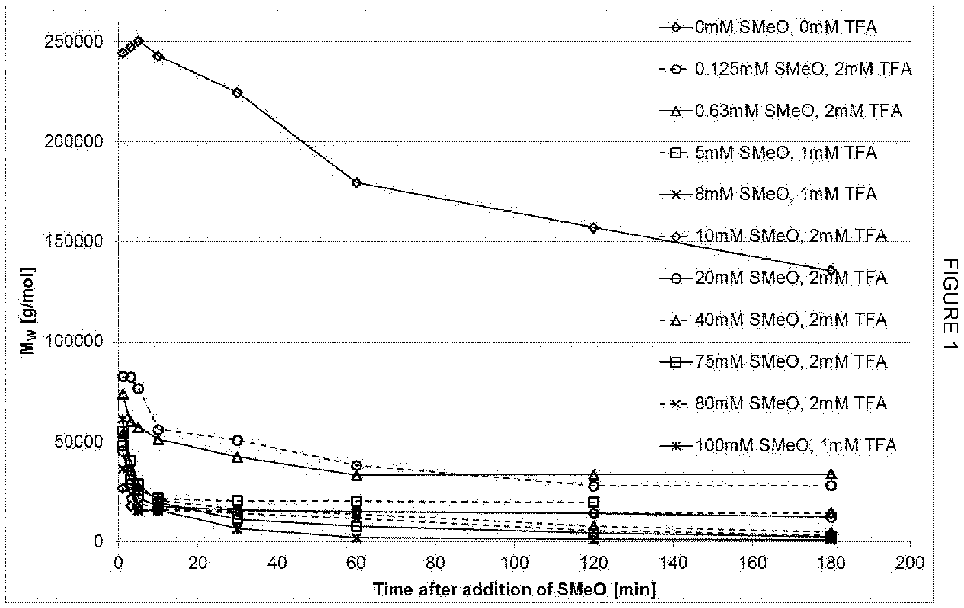

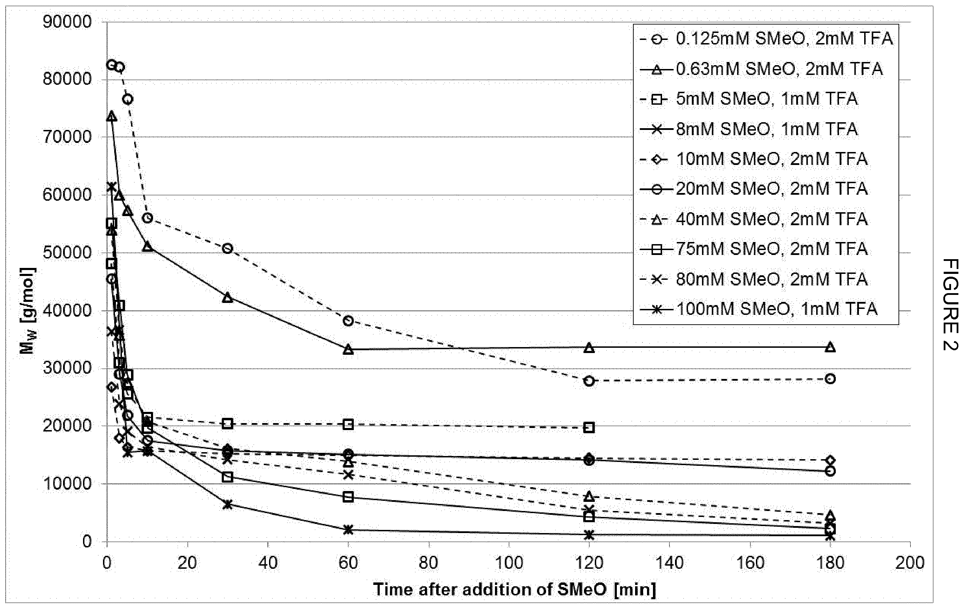

Figures 1 and 2 show the mass-average molecular mass (M_{W}) of PBCA formed in tetrahydrofuran (THF) after the addition of sodium methoxide (SMeO) as described in EXAMPLE 1.

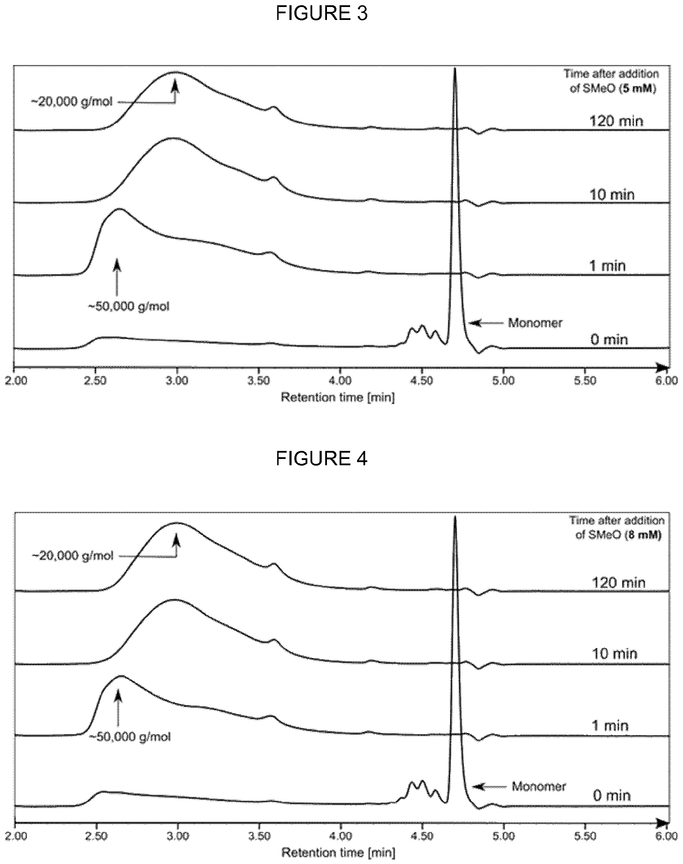

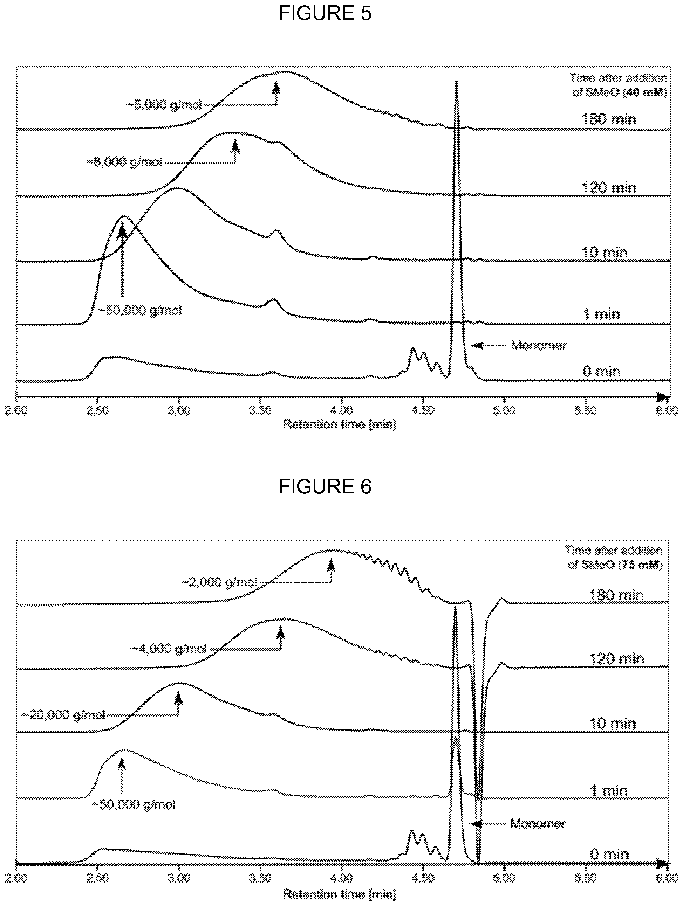

Figures 3, 4, 5 and 6 show Advanced Polymer Chromatography (APC) analyses of samples taken from PBCA preparations starting from 326 mM n-butyl 2-cyanoacrylate (BCA) and 1 or 2 mM trifluoroacetic acid (TFA) in THF 0, 1, 10, 120 or 180 min after addition of SMeO to a final concentration of 5, 8, 40 or 75 mM, respectively, as described in EXAMPLE 1.

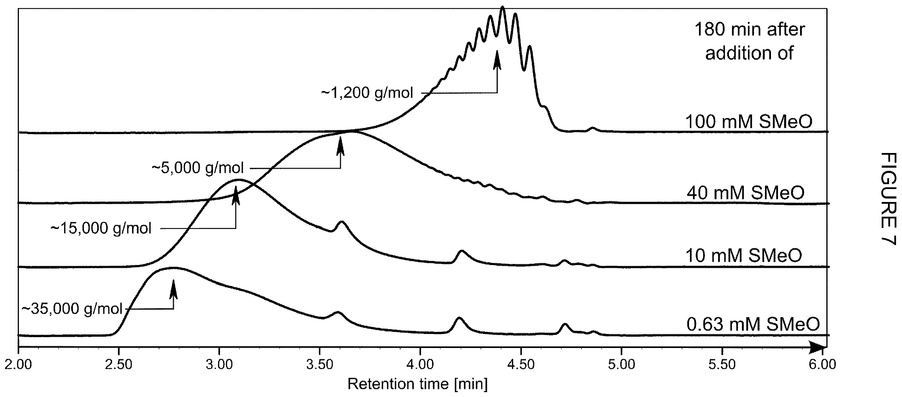

Figure 7 shows the APC analysis of samples taken from PBCA preparations 180 min after addition of SMeO to a final concentration of 0.63, 10, 40 or 100 mM, respectively, as described in EXAMPLE 1.

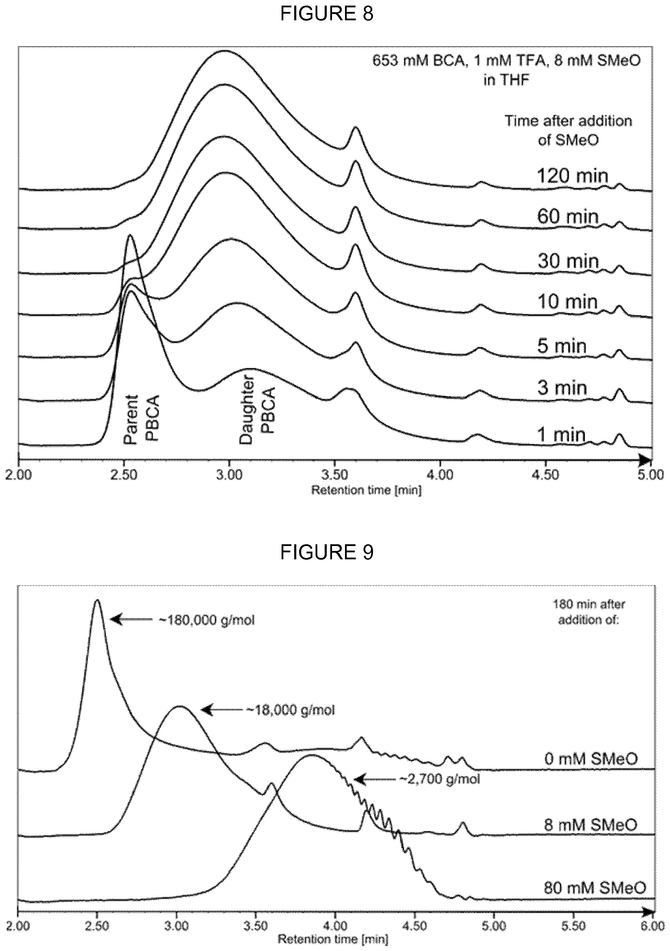

Figure 8 shows the APC analysis of samples taken from PBCA preparations starting from 653 mM n-butyl 2-cyanoacrylate (BCA), 8 mM SMeO and 1 mM trifluoroacetic acid (TFA) in THF 1, 3, 5, 10, 30, 60 and 120 min after addition of SMeO, respectively, as described in EXAMPLE 1.
Figure 9 shows the APC analysis of samples taken from PBCA preparations 180 min after addition of SMeO to a final concentration of 0, 8 or 80 mM, respectively, as described in EXAMPLE 2.

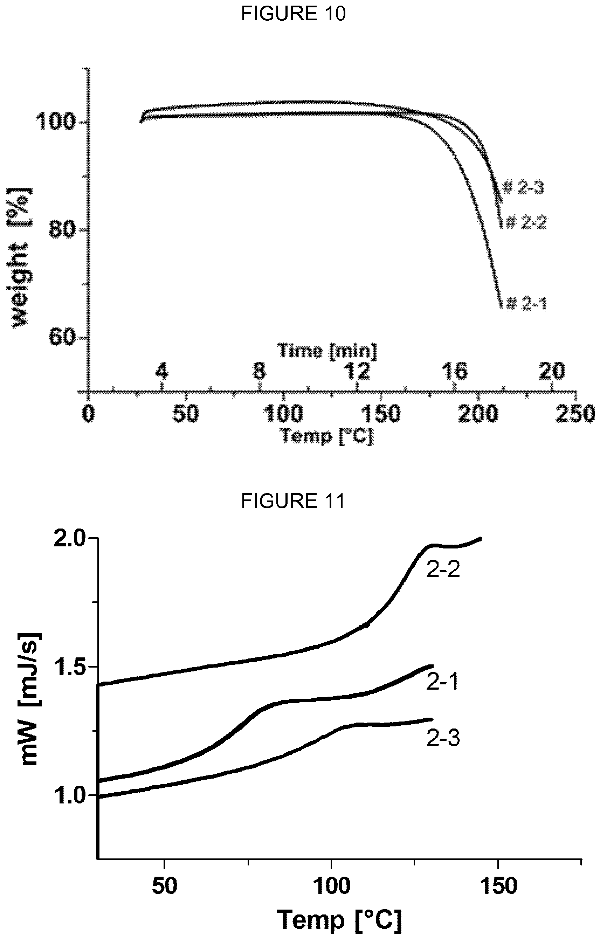

Figure 10 shows the results of the Thermal Gravimetric Analysis (TGA) of PBCA samples described in EXAMPLE 3 indicated as the weight loss of the sample over the temperature ("Temp").
Figure 11 shows the results of the analysis of PBCA samples *via* Differential Scanning Calorimetry (DSC) described in EXAMPLE 4 indicated as the heat flow rate (mW = mJ/s) over the temperature ("Temp").

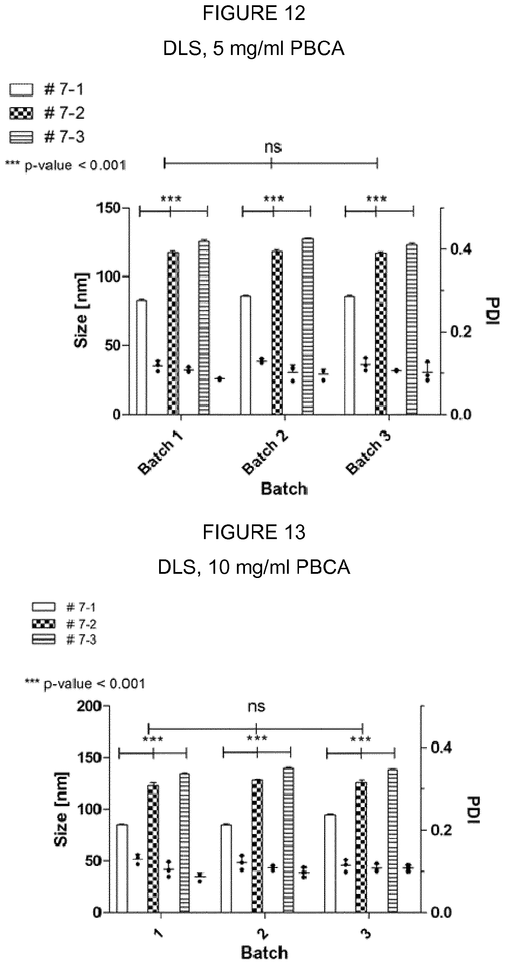

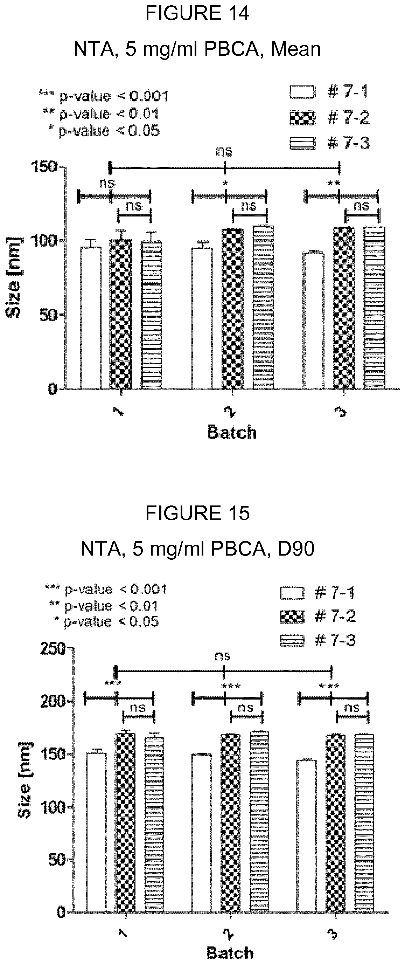

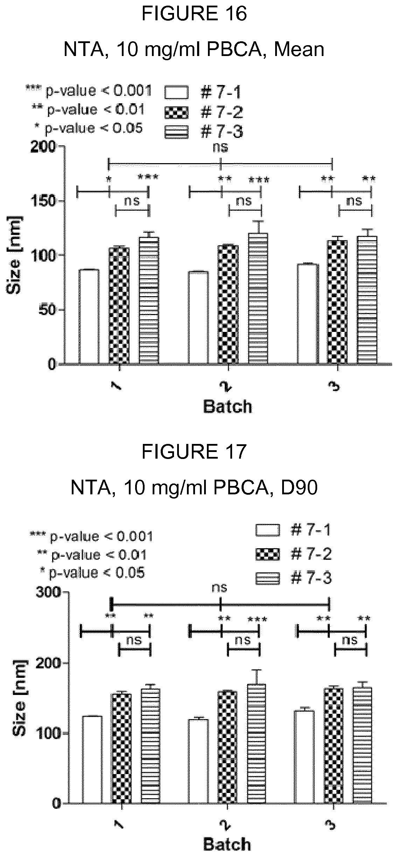

Figures 12, 13, 14, 15, 16 and 17 show the size of "empty" nanoparticles (prepared as described in EXAMPLE 7 from PBCA having a M_{W} of 2,618 g/mol ("# 7-1"), 17,162 g/mol ("# 7-2") or 173,422 g/mol ("# 7-3")) determined by dynamic light scattering (DLS) or nanoparticle tracking analysis (NTA), respectively. The experiments were performed in triplicates ("Batch 1", "Batch 2", "Batch 3"). No significant size variability (ns) was observed between the batches.

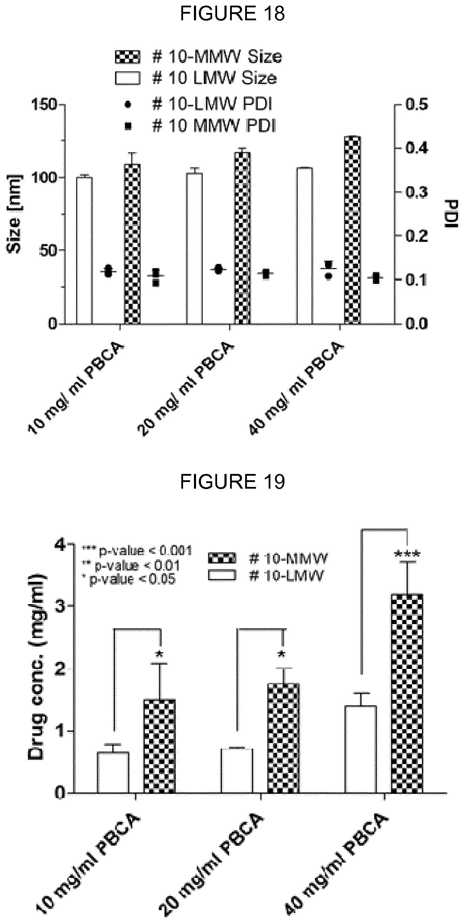

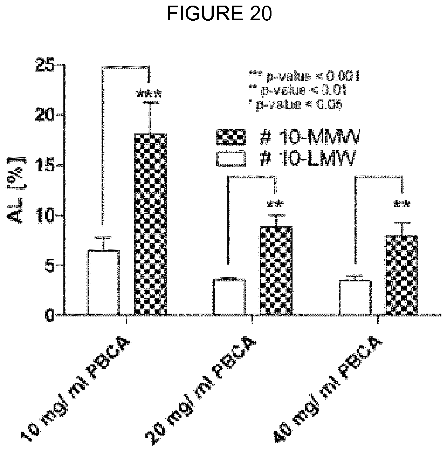

Figures 18, 19 and 20 show the size (determined *via* DLS, in nm), Budesonide concentration ("conc.", in mg/ml) and absolute drug loading ("AL", in %), respectively, of PBCA-based Budesonide-loaded nanoparticles prepared as described in EXAMPLE 10 using PBCA having a Mw of 12,760 g/mol ("# 10-MMW") or 2,613 g/mol ("# 10-LMW").

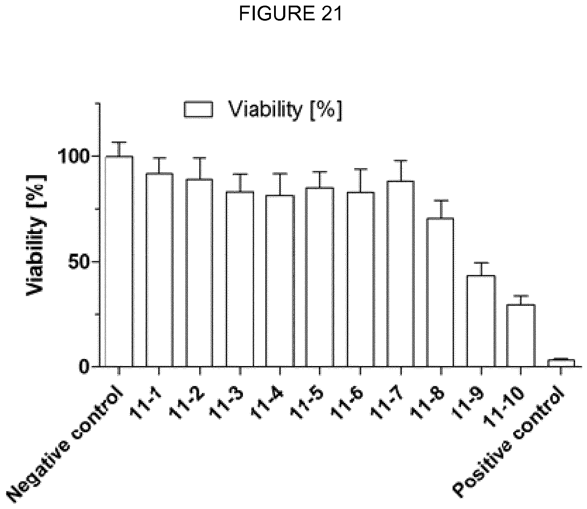

Figure 21 shows the viability of CACO-2 cells in the presence of only growth medium ("negative control", equals 100% viability), or growth medium containing 0.039 mM sodium cholate and 0.039 µM polysorbate 80 ("11-1"), 0.078 mM sodium cholate and 0.078 µM polysorbate 80 ("11-2"), 0.156 mM sodium cholate and 0.156 µM polysorbate 80 ("11-3"), 0.313 mM sodium cholate and 0.313 µM polysorbate 80 ("11-4"), 0.625 mM sodium cholate and 0.625 µM polysorbate 80 ("11-5"), 1.25 mM sodium cholate and 1.25 µM polysorbate 80 ("11-6"), 2.5 mM sodium cholate and 2.5 µM polysorbate 80 ("11-7"), 5.0 mM sodium cholate and 5.0 µM polysorbate 80 ("11-8"), 7.5 mM sodium cholate and 7.5 µM polysorbate 80 ("11-9") or 10 mM sodium cholate and 10 µM polysorbate 80 ("11-10"), respectively, or 100% DMSO ("positive control") determined as described in EXAMPLE 11.

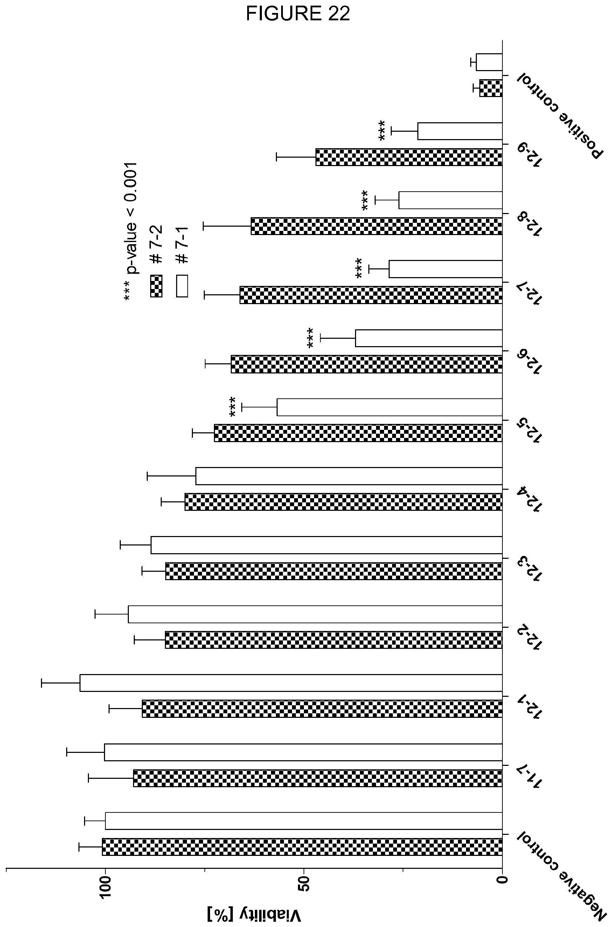

Figure 22 shows the viability of CACO-2 cells in the presence of only growth medium ("negative control", equals 100% viability), or growth medium containing 2.5 mM sodium cholate and 2.5 µM polysorbate 80 ("11-7"), or growth medium containing "empty" nanoparticles prepared with PBCA of a M_{W} of 2,618 g/mol ("# 7-1") or 17,162 g/mol ("# 7-2") at a final PBCA concentration of 10 µg/ml ("12-1"), 20 µg/ml ("12-2"), 39 µg/ml ("12-3"), 78 µg/ml ("12-4"), 156 µg/ml ("12-5"), 313 µg/ml ("12-6"), 625 µg/ml ("12-7"), 1250 µg/ml ("12-8") or 2500 µg/ml ("12-9"), respectively, or 100% DMSO ("positive control") determined as described in EXAMPLE 12.

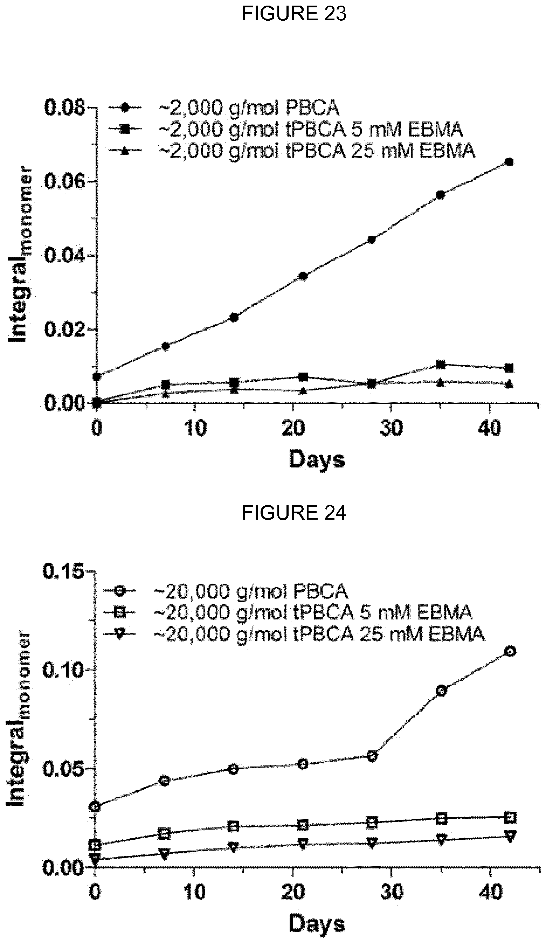

Figures 23 and 24 show the monomer release monitored in ¹H NMR measurements at 7.058 ppm (m) for PBCA and tPBCA, as described in EXAMPLE 15, where tPBCA is PBCA whose C-H acidic ends were terminated (capped) by reaction with ethyl 2-(bromomethyl) acrylate (EBMA). tPBCA was terminated using two different EBMA concentrations, 5 mM and 25 mM. PBCA and tPBCA having a M_{w} of ∼2,000 g/mol (Figure 23) or ∼20,000 g/mol (Figure 24) were stored for 7 weeks as solutions in CDCl₃.

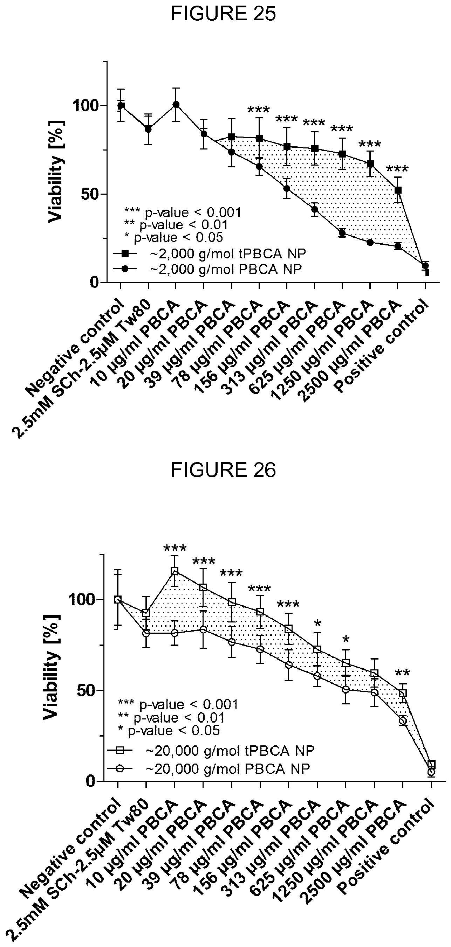

Figures 25 and 26 show the viability of CACO-2 cells after treatment with only growth medium ("negative control", equals 100% viability), or growth medium containing 2.5 mM sodium cholate and 2.5 µM polysorbate 80 ("2.5mM SCh-2.5µM Tw80"), or growth medium containing increasing concentrations of "empty" PBCA- and tPBCA-based nanoparticles (NP) having a Mw of ∼2,000 g/mol or ∼20,000 g/mol, respectively, or 100% DMSO ("positive control") determined as described in EXAMPLE 16 (mean ± SD; n=8). Figure 25 shows the viability results for ∼2,000 g/mol PBCA-based nanoparticles (circle) and ∼2,000 g/mol tPBCA-based nanoparticles (square). Figure 26 shows the viability results for ∼20,000 g/mol PBCA-based nanoparticles (open circle) and ∼20,000 g/mol tPBCA-based nanoparticles (open square). The specification of the concentrations on the x-axes relates both to PBCA and tPBCA, although denominated only as PBCA (e.g. "10µg/ml PBCA" stands for 10µg/ml PBCA or for 10µg/ml tPBCA) The statistical analysis was performed by two-way ANOVA.

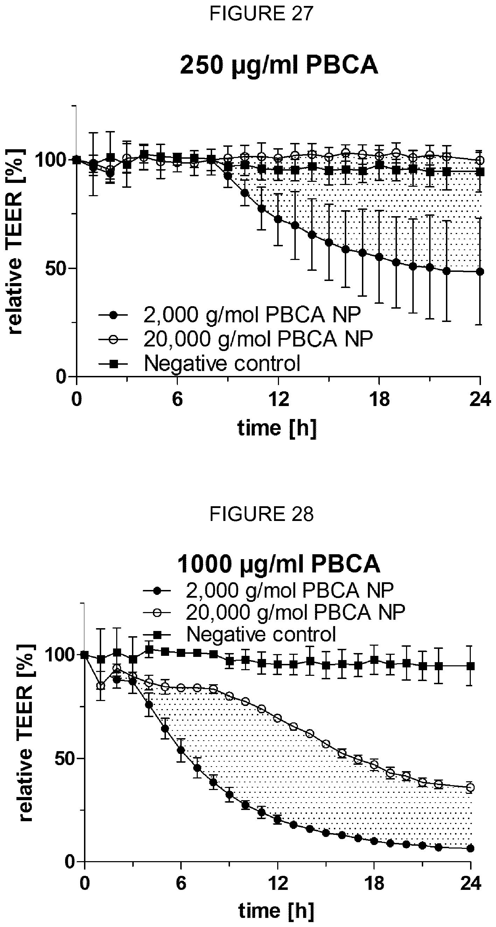

Figures 27 and 28 show the barrier integrity of CACO-2 cells as relative TEER value (mean ± SD; n=2) after treatment with PBCA-based nanoparticles (NP) in concentrations of 250 µg/ml (Figure 27) and 1000 µg/ml (Figure 28) comparing two PBCA M_{w} ranges ∼2,000 g/mol (circle), ∼20,000 g/mol (open square) and a negative control (square), as described in EXAMPLE 17.

### DETAILED DESCRIPTION OF THE INVENTION

Nanoparticles are solid submicron particles having a diameter within the nanometer range (i.e. between several nanometers to several hundred nanometers).

The nanoparticles of the invention are suitable for the encapsulation and delivery of cargo compounds (2) selected from pharmaceutically active agents, cosmetically active agents and nutritional supplements (herein also generally referred to as "cargo compounds"), in particular water-insoluble or poorly water-soluble (or "lipophilic") cargo compounds. Compounds are considered water-insoluble or poorly water-soluble if their solubility in water at 25°C (at pH 7.0) is 1 g/100 ml or less. In particular, the cargo compound encapsulated according to the invention has a solubility in water at 25°C and at pH 7.0 of 1 g/l or less, 0.5 g/l or less, preferably 0.1 g/l or less, 0.05 g/l or less, or especially 0.01 g/l or less.

The nanoparticles can protect the cargo compounds (2) on the way to the target site (e.g. the target cell) from degradation and/or modification by proteolytic and other enzymes and thus from the loss of their biological (e.g. pharmaceutical) activity. The invention is therefore also particularly useful for encapsulating cargo compounds which are susceptible to such enzymatic degradation and/or modification (e.g. polypeptides, peptides).

The terms "incorporated in" or "encapsulated by" [the nanoparticle or the matrix of the nanosphere] are used interchangeably herein. Likewise, the term "encapsulation" [of cargo compounds in nanoparticles] refers to the incorporation of the cargo compounds (2) in the nanoparticles. In contrast, molecules (such as antibodies) which are only attached to the surface of the nanoparticles are not "encapsulated by" or "incorporated in" the nanoparticles. Likewise, the expression [cargo compound-]"loaded" nanoparticles, as used herein, designates nanoparticles encapsulating said cargo compound.

The nanoparticles of the invention may have a mean particle size of less than 500 nm, less than 300 nm and in particular less than 200 nm, such as in the range of from 1-500 nm, 10-300 nm, preferably in the range of from 50-200 nm, and more preferably in the range of from 80-130 nm.

Unless indicated otherwise, the terms "size" and "diameter", when referring to a basically round object such as a nanoparticle (e.g. nanospheres or nanocapsules) or a droplet of liquid, are used interchangeably.

The physical scale "Mₙ", as used herein, refers to the number average molecular mass, also termed number average molecular weight, of all polymer chains in the sample and is defined by the equation: ${M}_{n} = \frac{\sum {N}_{x} ∗ {M}_{x}}{\sum {N}_{x}} .$

The summations over all different polymer chains from x=1 to x=∞ is multiplied with the specific molecular weight Mₓ of the polymer chain and divided by the sum of the number of molecules Nₓ of each molecular weight present in the sample.

The physical scale "M_{w}", as used herein, refers to the mass average molecular mass, also termed weight average molecular weight, as defined by the equation: ${M}_{w} = \frac{\sum {N}_{x} ∗ {M}_{x}^{2}}{\sum {N}_{x} ∗ {M}_{x}} .$

If not specified otherwise, the terms "molecular weight", "molecular mass" or "Mw" all refer to the mass average molar mass of a specific polymer.

The physical scale "Ð_{M}" (polydispersity index), as used herein, refer to the broadness of a molecular weight distribution and is defined by the equation: ${Ð}_{M} = \frac{{M}_{w}}{{M}_{n}} .$

The ratio between the mass average molecular mass and the number average molecular mass illustrates the distribution (polydispersity) of the polymer. A narrow molecular weight distribution has a value close to 1.

The term polydispersity or polydispersity index (Ð_{M}) as used in context with polymers has to be distinguished from the term polydispersity or polydispersity index (PDI) as used in context with nanoparticles: while in the former case the term describes a molecular weight distribution, in the latter case the term relates to the size distribution of the nanoparticles.

Size and polydispersity index (PDI) of a nanoparticle preparation can be determined, for example, by Dynamic Light Scattering (DLS, also known as Photon Correlation Spectroscopy or Quasi Elastic Light Scattering) and cumulant analysis according to the International Standard on Dynamic Light Scattering ISO13321 (1996) and ISO22412 (2008) which yields an average diameter (z-average diameter) and an estimate of the width of the distribution (PDI), e.g. using a Zetasizer device (Malvern Instruments, Germany; software version "Nano ZS"). Alternatively, the size of a nanoparticle preparation can be determined, for example, by nanoparticle tracking analysis (NTA) using a NanoSight LM10 device (Malvern Instruments, Germany) which yields a mean particle size as well as D10, D50 and D90 values (wherein D10, D50 and D90 designate diameters, with 10% of the particles having diameters lower than D10, 50% of the particles having diameters lower than D50, and 90% of the particles having diameters lower than D90).

The term "nanoparticles" is meant to include nanospheres as well as nanocapsules.

Nanospheres are nanoparticles comprising a polymeric matrix, wherein further components, such as cargo compounds (e.g. pharmaceutically or cosmetically active agents) can be incorporated (e.g. dissolved or dispersed). In addition to the cargo compounds, further ingredients can be incorporated (e.g. dissolved or dispersed), for example as described below.

The term "dispersed", as used herein, means that particles or droplets of the dispersed compound (the dispersed phase) are suspended throughout a continuous phase, i.e. form a chemically and physically heterogeneous mixture. In particular, the term is used to designate colloidal dispersions, wherein the dispersed phase particles have diameters in the range from about 1 nm to about 1 µm.

The term "dissolved", as used herein, means that the dissolved compound (solute) and the solvent, wherein the solute is dissolved, form a mixture (solution) that is chemically and physically uniform or homogenous throughout or consists of one phase.

According to a particular embodiment, at least 50%, in particular at least 70%, at least 80%, at least 90% or, preferably at least 95% or 100% of the cargo compound(s) (2) in the nanosphere is dissolved in the polymeric matrix of the nanosphere.

According to another particular embodiment, at least 50%, in particular at least 70%, at least 80%, at least 90% or, preferably at least 95% or 100% of the cargo compound(s) (2) in the nanosphere is present in a crystalline state.

According to another particular embodiment, at least 50%, in particular at least 70%, at least 80%, at least 90% or, preferably at least 95% or 100% of the cargo compound(s) (2) in the nanosphere is present in a semi-crystalline state.

According to another particular embodiment, at least 50%, in particular at least 70%, at least 80%, at least 90% or, preferably at least 95% or 100% of the cargo compound(s) (2) in the nanosphere is present in an amorphous state.

Nanocapsules are nanoparticles which have a core-shell structure, i.e. a core containing cargo compounds (e.g. pharmaceutically or cosmetically active agents) that is surrounded by a polymeric shell.

In the core of the nanocapsules of the invention, the cargo compound(s) (2) may be liquid or in the form of a liquid (e.g. aqueous or oily) solution or dispersion, although this is generally not preferred. Rather, according to one embodiment, at least 50%, in particular at least 70%, at least 80%, at least 90% or, preferably at least 95% or 100% of the cargo compound(s) (2) in the nanocapsule core is present in an undissolved solid form, such as an amorphous, semi-crystalline or crystalline state, or a mixture thereof.

According to a particular embodiment, at least 50%, in particular at least 70%, at least 80%, at least 90% or, preferably at least 95% or 100% of the cargo compound(s) (2) in the nanocapsule core is present in a crystalline state.

According to another particular embodiment, at least 50%, in particular at least 70%, at least 80%, at least 90% or, preferably at least 95% or 100% of the cargo compound(s) (2) in the nanocapsule core is present in a semi-crystalline state.

According to another particular embodiment, at least 50%, in particular at least 70%, at least 80%, at least 90% or, preferably at least 95% or 100% of the cargo compound(s) (2) in the nanocapsule core is present in an amorphous state.

The nanoparticles of the invention can have an advantageously high load of cargo compound(s). Thus, the nanoparticle of the invention can comprise at least 5 wt-%, at least 10 wt-%, at least 20 wt-%, at least 40 wt-%, at least 50 wt-%, at least 60 wt-%, at least 70 wt-%, preferably at least 80 wt-%, more preferably at least 90 wt-%, at least 95 wt-%, most preferably at least 99 wt-% or at least 99.9 wt-% and up to 99.9 wt-%, up to 99.95 wt-% or, preferably, up to 99.99 wt-% of the cargo compound(s) (2) relative to the total weight of the (e.g. matrix-forming or shell-forming) polymer(s) (1) and cargo compound(s) (2) of the nanoparticle.

The term "cyanoacrylates", as used herein, refers to compounds of the general formula (I) 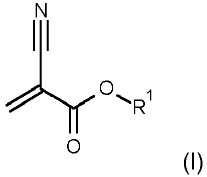 wherein R¹ is C₁-C₁₀-alkyl or C₁-C₆-alkoxy-C₁-C₁₀-alkyl.

The term "C₁-C₃-alkyl", as used herein, refers to saturated aliphatic hydrocarbon radicals which may be linear or branched and which have from 1 to 3 carbon atoms. "C₁-C₆-alkyl", as used herein, refers to saturated aliphatic hydrocarbon radicals which may be linear or branched and which have from 1 to 6 carbon atoms. "C₁-C₈-alkyl", as used herein, refers to saturated aliphatic hydrocarbon radicals which may be linear or branched and which have from 1 to 8 carbon atoms. "C₁-C₁₀-alkyl", as used herein, refers to saturated aliphatic hydrocarbon radicals which may be linear or branched and which have from 1 to 10 carbon atoms. Examples for C₁-C₃-alkyl are methyl, ethyl, propyl and isopropyl. Examples for C₁-C₆-alkyl are, in addition to those listed above for C₁-C₃-alkyl, n-butyl, isobutyl, sec-butyl, tert-butyl, n-pentyl, 2-pentyl, 2-methylbutyl, 3-methylbutyl, 1,2-dimethylpropyl, 1,1-dimethylpropyl, 2,2-dimethylpropyl, 1-ethylpropyl, n-hexyl, 2-hexyl, 2-methylpentyl, 3-methylpentyl, 4-methylpentyl, 1,2-dimethylbutyl, 1,3 dimethylbutyl, 2,3-dimethylbutyl, 1,1-dimethylbutyl, 2,2-dimethylbutyl, 3,3 dimethylbutyl, 1,1,2-trimethylpropyl, 1,2,2-trimethylpropyl, 1-ethylbutyl, 2-ethylbutyl, 1-ethyl-2-methylpropyl and the like. Examples for C₁-C₈-alkyl are, in addition to those listed above for C₁-C₆-alkyl, n-heptyl, 1-methylhexyl, 2-methyl¬hexyl, 1-ethylpentyl, 2-ethylpentyl, 1-propylbutyl, 1-ethyl-2-methylbutyl, n-octyl, isooctyl, 2 ethylhexyl and the like. Examples for C₁-C₁₀-alkyl are, in addition to those listed above for C₁-C₈-alkyl, n-nonyl, isononyl, 2-propylhexyl, n-decyl, isodecyl, 2-propyl¬heptyl, and the like. Preferably, "linear or branched C₁-C₁₀-alkyl" refers to linear or branched alkyl groups having 1 to 6 carbon atoms (C₁-C₆-alkyl).

In connection with the present invention, the term "C₂-C₁₀-alkenyl radical" refers to unsaturated aliphatic hydrocarbon radicals which have one C-C double bond, as well as to unsaturated aliphatic hydrocarbon radicals which have two or more non-cumulated C-C double bonds.

Accordingly, the term "C₂-C₁₀-alkenyl radical comprising at least one double bond of which at least one is terminal", as used herein, refers to linear or branched unsaturated aliphatic hydrocarbon radicals which have from 2 to 10 carbon atoms and which have 1, 2 or 3 C-C double bonds of which at least one is a terminal double bond.

"Terminal" in this context refers to a double bond in which one of the two olefinic carbon atoms is unsubstituted (corresponding to =CH₂).

Preferably, the term "C₂-C₁₀-alkenyl radical comprising at least one double bond of which at least one is terminal" refers to linear or branched unsaturated aliphatic hydrocarbon radicals which have from 1 to 6 carbon atoms and which have 1 or 2 C-C double bonds of which one double bond is a terminal double bond ("C₂-C₆-alkenyl radical comprising at least one double bond of which at least one is terminal"). These include, for example, ethenyl, 2-propene-1-yl, 1-propene-2-yl, 3-butene-1-yl, 1-butene-2-yl, 1,3-butadiene-1-yl, 2-methyl-2-propene-1-yl, 2-methylenepropane-1-yl, 4-pentene-1-yl, 1-pentene-2-yl, 2-methylenebutane-1-yl, 1,4-pentadiene-1-yl, 2,4-pentadiene-1-yl, 2-methyl-3-butene-1-yl, 3-methyl-3-butene-1-yl, 2-methyl-1,3-butadiene-1-yl, 5-hexene-1-yl, 1-hexene-2-yl, 2-methylenepentane-1-yl, 3-methylenepentane-1-yl, 1,5-hexadiene-1-yl, 2,5-hexadiene-1-yl, 3,5-hexadiene-1-yl, 2-methyl-4-pentene-1-yl, 3-methyl-4-pentene-1-yl, 4-methyl-4-pentene-1-yl, and the like.

The term "C₁-C₃-alkoxy", as used herein, refers to linear or branched alkyl groups having from 1 to 3 carbon atoms, as defined above, which are bound to the remainder of the molecule via an oxygen atom. "C₁-C₆-Alkoxy", as used herein, refers to linear or branched alkyl groups having from 1 to 6 carbon atoms, as defined above, which are bound to the remainder of the molecule via an oxygen atom. "C₁-C₁₀-Alkoxy", as used herein, refers to linear or branched alkyl groups having from 1 to 10 carbon atoms, as defined above, which are bound to the remainder of the molecule via an oxygen atom. Examples for C₁-C₃-alkoxy are methoxy, ethoxy, n-propoxy and 1-methylethoxy (isopropoxy). Examples for C₁-C₆-alkoxy are, in addition to those mentioned above for C₁-C₃-alkoxy, butoxy, 1-methylpropoxy (sec-butoxy), 2-methylpropoxy (isobutoxy), 1,1-dimethylethoxy (tert-butoxy), n-pentoxy, 1-methylbutoxy, 2-methylbutoxy, 3-methylbutoxy, 1,2-dimethylpropoxy, 1-ethylpropoxy, n-hexyloxy, 1-methylpentoxy, 2-methylpentoxy, 3-methylpentoxy, 4-methylpentoxy, 1,2-dimethylbutoxy, 1-ethylbutyoxy, 2-ethylbutoxy, and the like. Examples for C₁-C₁₀-alkoxy are, in addition to those mentioned above for C₁-C₆-alkoxy, n-heptoxy, 1-methylhexoxy, 2-methyl¬hexoxy, 1-ethylpentoxy, 2-ethylpentoxy, 1-propylbutoxy, 1-ethyl-2-methylbutoxy, n-octyloxy, isooctyloxy, 2 ethylhexoxy, n-nonyloxy, isononyloxy, 2-propylhexoxy, n-decyloxy, isodecyloxy, 2-propyl¬heptoxy, and the like

The term "C₁-C₃-alkoxy-C₁-C₃-alkyl", as used herein, refers to a C₁-C₃-alkyl radical, as defined above, in which one hydrogen atom is replaced by a C₁-C₃-alkoxy radical, as defined above. The term "C₁-C₆-alkoxy-C₁-C₁₀-alkyl", as used herein, refers to a C₁-C₁₀-alkyl radical, as defined above, in which one hydrogen atom is replaced by a C₁-C₆-alkoxy radical, as defined above. Examples include methoxymethyl, ethoxymethyl, propoxymethyl, iso-propoxymethyl, n-butoxymethyl, iso-butoxymethyl, sec.-butoxymethyl, tert.-butoxymethyl, pentoxymethyl, iso-pentoxymethyl, hexoxymethyl, 1-methoxyethyl, 1-ethoxyethyl, 1-propoxyethyl, 1-iso-propoxyethyl, 1-n-butoxyethyl, 1-iso-butoxyethyl, 1-sec.-butoxyethyl, 1-tert.-butoxyethyl, 1-n-pentoxyethyl, 1-iso-pentoxyethyl, 1-n-hexoxyethyl, 2-methoxyethyl, 2-ethoxyethyl, 2-propoxyethyl, 2-iso-propoxyethyl, 2-n-butoxyethyl, 2-iso-butoxyethyl, 2-sec.-butoxyethyl, 2-tert.-butoxyethyl, 2-n-pentoxyethyl, 2-iso-pentoxyethyl, 2-n-hexoxyethyl, 1-methoxypropyl, 1-ethoxypropyl, 1-propoxypropyl, 1-iso-propoxypropyl, 1-n-butoxypropyl, 1-iso-butoxypropyl, 1-sec.-butoxypropyl, 1-tert.-butoxypropyl, 1-n-pentoxypropyl, 1-iso-pentoxypropyl, 1-n-hexoxypropyl, 2-methoxypropyl, 2-ethoxypropyl, 2-propoxypropyl, 2-iso-propoxypropyl, 2-n-butoxypropyl, 1-iso-butoxypropyl, 2-sec.-butoxypropyl, 2-tert.-butoxypropyl, 2-n-pentoxypropyl, 2-iso-pentoxypropyl, 2-n-hexoxypropyl, 3-methoxypropyl, 3-ethoxypropyl, 3-propoxypropyl, 3-iso-propoxypropyl, 3-n-butoxypropyl, 3-iso-butoxypropyl, 3-sec.-butoxypropyl, 3-tert.-butoxypropyl, 3-n-pentoxypropyl, 3-iso-pentoxypropyl, 3-n-hexoxypropyl, 1-methoxybutyl, 1-ethoxybutyl, 1-propoxybutyl, 1-iso-propoxybutyl, 1-n-butoxybutyl, 1-iso-butoxybutyl, 1-sec.-butoxybutyl, 1-tert.-butoxybutyl, 1-n-pentoxybutyl, 1-iso-pentoxybutyl, 1-n-hexoxybutyl, 2-methoxybutyl, 2-ethoxybutyl, 2-propoxybutyl, 2-iso-propoxybutyl, 2-n-butoxybutyl, 2-iso-butoxybutyl, 2-sec.-butoxybutyl, 2-tert.-butoxybutyl, 2-n-pentoxybutyl, 2-iso-pentoxybutyl, 2-n-hexoxybutyl, 3-methoxybutyl, 3-ethoxybutyl, 3-propoxybutyl, 3-iso-propoxybutyl, 3-n-butoxybutyl, 3-iso-butoxybutyl, 3-sec.-butoxybutyl, 3-tert.-butoxybutyl, 3-n-pentoxybutyl, 3-iso-pentoxybutyl, 3-n-hexoxybutyl, 4-methoxybutyl, 4-ethoxybutyl, 4-propoxybutyl, 4-iso-propoxybutyl, 4-n-butoxybutyl, 4-iso-butoxybutyl, 4-sec.-butoxybutyl, 4-tert.-butoxybutyl, 4-n-pentoxybutyl, 4-iso-pentoxybutyl, 4-n-hexoxybutyl, 5-methoxypentyl, 5-ethoxypentyl, 5-propoxypentyl, 5-iso-propoxypentyl, 5-n-butoxypentyl, 5-iso-butoxypentyl, 5-sec.-butoxypentyl, 5-tert.-butoxypentyl, 5-n-pentoxypentyl, 5-iso-pentoxypentyl, 5-n-hexoxypentyl, 6-methoxyhexyl, 6-ethoxyhexyl, 6-propoxyhexyl, 6-iso-propoxyhexyl, 6-n-butoxyhexyl, 6-iso-butoxyhexyl, 6-sec.-butoxyhexyl, 6-tert.-butoxyhexyl, 6-n-pentoxyhexyl, 6-iso-pentoxyhexyl, 6-n-hexoxyhexyl, and the like, as well as isomers thereof.

The term "C₁-C₁₀-alkylcarbonyl", as used herein, refers to linear or branched C₁-C₁₀-alkyl radicals, as defined above, which are bound to the remainder of the molecule via a carbonyl group (-C(=O)-C₁-C₁₀-alkyl). Examples are methylcarbonyl (acetyl), ethylcarbonyl (propionyl), propylcarbonyl, isopropylcarbonyl, butylcarbonyl and the like.

The term "C₁-C₁₀-alkoxycarbonyl", as used herein, refers to linear or branched C₁-C₁₀-alkoxy radicals, as defined above, which are bound to the remainder of the molecule via a carbonyl group (-C(=O)-O-C₁-C₁₀-alkyl). Examples are methoxycarbonyl, ethoxycarbonyl, propoxycarbonyl, isopropoxycarbonyl, butoxycarbonyl and the like
The term "C₁-C₃-alkanol", as used herein, refers to methanol, ethanol, n-propanol or iso-propanol, preferably to methanol, ethanol or iso-propanol.

More preferably the term "C₁-C₃-alkanol", as used herein, refers to methanol or ethanol, in particular to methanol.

"Alkoxides" are anions of alkanols of formula R-O-, where R is an alkyl group. If not specified otherwise, the alkyl group is C₁-C₄-alkyl and the alkoxide thus C₁-C₄-alkoxide. Examples for C₁-C₄-alkoxide are methoxide, ethoxide, propoxide, isopropoxide, butoxide, sec-butoxide, isobutoxide and tert-butoxide.

The polymers (1) on which the nanoparticles of the present invention are based, i.e. the matrix-forming polymers of the nanospheres of the invention and the shell-forming polymers of the nanocapsules of the invention, comprise monomeric constituents selected from C₁-C₁₀-alkyl cyanoacrylates, such as C₁-C₈-alkyl cyanoacrylates, and C₁-C₆-alkoxy-C₁-C₁₀-alkyl cyanoacrylates, such as C₁-C₃-alkoxy-C₁-C₃-alkyl cyanoacrylates. Examples of said monomeric constituents include, but are not limited to, methyl 2-cyanoacrylate, 2-methoxyethyl 2-cyanoacrylate, ethyl 2-cyanoacrylate, n-butyl 2-cyanoacrylate, isobutyl 2-cyanoacrylate, *sec*-butyl 2-cyanoacrylate, tert-butyl 2-cyanoacrylate, n-hexyl-2-cyanoacrylate, isohexyl-cyanoacrylate and n-octyl 2-cyanoacrylate, wherein ethyl-2-cyanoacrylate, n-octylcyanoacrylate and butyl 2-cyanoacrylates (in particular n-butyl-2-cyanoacrylate) are preferred, and butyl 2-cyanoacrylate such as n-butyl-2-cyanoacrylate is particularly preferred.

The C₁-C₁₀-alkyl cyanoacrylates and/or C₁-C₆-alkoxy-C₁-C₁₀-alkyl cyanoacrylates described herein are the main monomeric constituent of the polymers (1) comprised by the nanoparticles of the present invention, or the polymers (1) prepared by or used in the methods of the present invention. Most preferably, said polymers do not comprise monomeric constituents of a type different. The term "main monomeric constituent", as used herein, designates a monomeric constituent that makes up at least 80 wt-%, at least 90 wt-%, at least 95 wt-%, at least 98 wt-%, preferably at least 99 wt-% and up to 100 wt-% of the polymer (1).

The polymers (1) of the nanoparticles of the present invention may be selected from poly(C₁-C₁₀-alkyl cyanoacrylates), such as poly(C₁-C₈-alkyl cyanoacrylates), and poly(C₁-C₆-alkoxy-C₁-C₁₀-alkyl cyanoacrylates), such as poly(C₁-C₃-alkoxy-C₁-C₃-alkyl cyanoacrylates). Examples of useful polymers (1) include, but are not limited to, poly(methyl 2-cyanoacrylates), poly(2-methoxyethyl 2-cyanoacrylates), poly(ethyl 2-cyanoacrylates), poly(n-butyl 2-cyanoacrylates), poly(isobutyl 2-cyanoacrylates), poly(*sec*-butyl 2-cyanoacrylates), poly(*tert*-butyl 2-cyanoacrylates), poly(n-hexyl-2-cyanoacrylates), poly(isohexyl-cyanoacrylate) and poly(n-octyl 2-cyanoacrylates), wherein poly(ethyl-2-cyanoacrylates), poly(n-octylcyanoacrylates) and poly(butyl 2-cyanoacrylates) (in particular poly(n-butyl-2-cyanoacrylates)) are preferred, and poly(butyl 2-cyanoacrylates) such as poly(n-butyl-2-cyanoacrylates) are particularly preferred.

According to particularly preferred embodiments, the nanoparticles of the present invention comprise only one type of poly(C₁-C₁₀-alkyl cyanoacrylate) or poly(C₁-C₆-alkoxy-C₁-C₁₀-alkyl cyanoacrylate) as described herein.

The polymers (1) comprised by the nanoparticles of the invention and used in the method for preparing nanoparticles according to the present invention:
- comprise from 60 to 330, preferably from 60 to 200, more preferably from 60 to 165, most preferably from 65 to 130, monomeric constituents selected from C₁-C₁₀-alkyl cyanoacrylates and C₁-C₆-alkoxy-C₁-C₁₀-alkyl cyanoacrylates as described herein, and
- have a dispersity (Ð_{M}) of less than 3.0, preferably less than 2.5, more preferably less than 2.0, such as a dispersity in the range of from about 1.0-3.0, in particular from 1.2-2.5, especially from 1.5-2.0.

Such polymers (1) can be prepared by the method for preparing a polymer having a defined degree of polymerization described herein.

The degree of polymerization (number of monomeric constituents) determines the molecular weight of the polymer. Thus, a poly(butyl cyanoacrylate) comprising from 60 to 330 butyl cyanoacrylate monomeric constituents has a molecular weight of from about 9,200 to about 50,500 g/mol.

In particular embodiments of the nanoparticles and methods of the invention, where the polymer is poly(butyl cyanoacrylate) as described herein, the poly(butyl cyanoacrylate) has a dispersity (Ð_{M}) as described herein and a mass-average molecular mass (Mw) in the range of from about 9,200 g/mol to about 50,500 g/mol, preferably from about 9,200 g/mol to about 30,600 g/mol, more preferably from about 9,200 g/mol to about 25,200 g/mol and most preferably from about 10,000 g/mol to about 19,900 g/mol.

The mass-average molecular mass (M_{W}) and the number-average molecular mass (Mₙ) of a polymer (and thus also the dispersity Ð_{M} = M_{W}/ Mₙ thereof) can be determined, for example, by Gel Permeation Chromatography (GPC), also termed Size Exclusion Chromatography (SEC), or by Advanced Polymer Chromatography (APC, systems provided by Waters, Janco et al., J Sep Sci 36(17):2718-27, 2013) using a molecular mass standard (e.g. polystyrene molecular mass standards having peak molecular masses in the range of from 1,210,000 to 474 g/mol) under condition such as, for example, mobile phase: 100% THF, flow: 1 ml/min, 3 successively arranged columns, each packed with trimethyl silane-modified particles of ethylene-bridged hybrid material (polyethoxysilane, cf. US 6,686,035): column 1 (e.g. ACQUITY APC XT 200): 4.6 mm x 150 mm, 200 Å pore size , 2.5 µm mean particle size; each of columns 2 and 3 (e.g. ACQUITY APC XT 45): 4.6 mm x 1500mm, 45 Å pore size, 1.7 µm mean particle size.

The number of monomeric constituents (i.e. the degree of polymerization) of a polymer can be determined as known in the art. For example, for a homopolymer, where only one type of monomeric constituent is present, the number-average degree of polymerization (DPₙ) can be calculated from the Mₙ of a homopolymer and the molecular mass of the monomeric constituent Mo as DPₙ=Mₙ/M₀. Likewise, the mass-average degree of polymerization (DP_{W}) can be calculated from the M_{W} of a homopolymer and the molecular mass of the monomeric constituent Mo as DP_{W}=M_{W}/M₀.

The nanoparticles of the invention are prepared from pre-synthesized and, if required, purified polymer(s) (1). The nanoparticles can therefore be essentially free of monomers, i.e. essentially free of monomeric C₁-C₁₀-alkyl cyanoacrylates, C₁-C₆-alkoxy-C₁-C₁₀-alkyl cyanoacrylates and salts thereof.

The term "essentially free of monomers" refers to amounts of less than 10 wt-%, preferably less than 5 wt-%, more preferably less than 2 wt-% and in particular less than 1 wt-%, for example less than 0.01 wt-% or less than 0.05 wt-%, monomers relative to the total weight of the polymer(s) (1).

The term "about" is understood by persons of ordinary skill in the art in the context in which it is used herein. In particular, "about" is meant to refer to variations of ±20%, ±10%, preferably ±5%, more preferably ±1%, and still more preferably ±0.1%.

The nanoparticles of the invention can comprise one or more than one nanoparticle-stabilizing agent selected from bile acids (e.g. cholic acid, taurocholic acid, glycocholic acid, deoxycholic acid, lithocholic acid, chenodeoxycholic acid, dehydrocholic acid, ursodeoxycholic acid, hyodeoxycholic acid and hyocholic acid), salts (e.g. sodium, potassium or calcium salts) of bile acids, and mixtures thereof. The nanoparticle-stabilizing agent allows for the formation of stable nanoparticles even where the nanoparticles are highly drug-loaded, i.e. the amount of polymers (1) is very low. Preferably, the nanoparticle-stabilizing agent is selected from cholic acid, one or more than one salt of cholic acid, and mixtures thereof. According to a particularly preferred embodiment, the nanoparticle-stabilizing agent is sodium cholate.

The one or more than one nanoparticle-stabilizing agent is typically present in an amount of from 3 to 36 wt-% relative to the total weight of the polymer(s) (1) and cargo compound(s) (2) of the nanoparticle.

The nanoparticle of the invention can (further) comprise one or more than one uptake mediator selected from polyoxyethylene sorbitan fatty acid esters. Said uptake mediator(s) can facilitate the transport of the nanoparticles across barriers within the organism, in particular across the blood-brain barrier. It is hypothesized that polyoxyethylene sorbitan fatty acid esters such as polyoxyethylene (20) sorbitan monooleate (polysorbate 80) facilitate an attraction of specific plasma proteins, such as ApoE, which play a key role in the receptor-mediated uptake of compounds by brain capillary cells (Kreuter et al., J Drug Target 10(4):317-25, 2002).

Examples of uptake mediators include polyoxyethylene sorbitan monoesters and triesters with monounsaturated or, in particular, saturated fatty acids. Examples of particular fatty acids include, but are not limited to, C₁₁-C₁₈-fatty acids such as lauric acid, palmitic acid, stearic acid and, in particular, oleic acid. The polyoxyethylene sorbitan fatty acid esters may comprise up to 90 oxyethylene units, for example 15-25, 18-22 or, preferably, 20 oxyethylene units. The uptake mediator(s) is/are preferably selected from polyoxyethylene sorbitan fatty acid esters having an HLB value in the range of about 13-18, in particular about 16-17. Expediently, the uptake mediator(s) used in the nanoparticles of the invention are selected from officially approved food and/or drug additives such as, for example, polysorbate 20 (E432), polysorbate 40 (E434), polysorbate 60 (E435), polysorbate 65 (E436) and, in particular, polysorbate 80 (E433). Preferably, the uptake mediator is polysorbate 80.

The one or more than one uptake mediator is typically present in an amount of from 0.001 to 0.1 wt-% relative to the total weight of the polymer(s) (1) and cargo compound(s) (2) of the nanoparticle.

The nanoparticle of the invention can (further) comprise one or more than one sorbitan fatty acid ester. Said sorbitan fatty acid(s) can facilitate the formation of nanoparticles having a reduced size, e.g. a diameter of less than 200 nm.

Examples of suitable sorbitan fatty acid esters include, but are not limited to, sorbitan monoesters of monounsaturated or, in particular, saturated C₁₁-C₁₈-fatty acids such as lauric acid, palmitic acid, stearic acid and, in particular, oleic acid. According to a preferred embodiment, the sorbitan fatty acid ester is sorbitan monooleate.

The nanoparticle of the invention, in particular its outer layer, can (further) comprise one or more than one amphiphilic lipids that, for example, can serve as a detectable label, is linked to a targeting compound or carries a linker allowing for the attachment of, for example, targeting or labelling compounds.

The term "amphiphilic lipid", as used herein, refers to a molecule comprising a hydrophilic part and a hydrophobic part. Generally, the hydrophobic part of an amphiphilic lipid comprises one or more than one linear or branched saturated or unsaturated hydrocarbon chain having from 7 to 29 carbon atoms (i.e. is derived from a C₈-C₃₀ fatty acid). Examples of suitable amphiphilic lipids for use in the nanoparticles of the invention include naturally occurring or synthetic phospholipids, cholesterols, lysolipids, sphingomyelins, tocopherols, glucolipids, stearylamines and cardiolipins. Further examples include esters and ethers of one or more than one (e.g. one or two) fatty acid with a hydrophilic compound such as a sugar alcohol (e.g. sorbitan) or saccharide (such as a mono-, di- or trisaccharide, e.g. saccharose). The amphiphilic lipid used in the nanoparticles of the invention expediently carries a functional moiety, such as a linker, detectable and/or targeting moiety. Said moiety is preferably covalently coupled to the hydrophilic part of the amphiphilic lipid, optionally via a spacer. Such spacer may comprise, or essentially consist of, a polyoxyethylene chain.

The amphiphilic lipid used in the nanoparticles of the invention is preferably a phospholipid that carries a functional moiety selected from a linker, detectable and/or targeting moiety as described herein.

The term "lipid", as used herein, refers to a fat, oil or substance containing esterified fatty acids present in animal fats and in plant oils. Lipids are hydrophobic or amphiphilic molecules mainly formed of carbon, hydrogen and oxygen and have a density lower than that of water. Lipids can be in a solid state at room temperature (25°C), as in waxes, or liquid as in oils.

The term "fatty acid", as used herein, refers to an aliphatic monocarboxylic acid having a, generally linear, saturated or unsaturated hydrocarbon chain and at least 4 carbon atoms, typically from 4 to 30 carbon atoms. Natural fatty acids mostly have an even number of carbon atoms and from 4 to 30 carbon atoms. Long chain fatty acids are those having from 14 to 22 carbon atoms; and very long chain fatty acids are those having more than 22 carbon atoms.

The term "phospholipid", as used herein, refers to a lipid having a phosphate group, in particular a phosphoglyceride. Phospholipids comprise a hydrophilic part including the phosphate group and a hydrophobic part formed by (typically two) fatty acid hydrocarbon chains. Particular phospholipids include phosphatidylcholine, phosphatidylethanolamines (e.g. 1,2-distearoyl-sn-glycero-3-phosphoethanolamine), phosphatidylinositol, phosphatidylserine and sphingomyelin which carry a functional moiety as described herein.

According to one embodiment, the nanoparticle of the invention comprises one or more than one amphiphilic lipid, wherein said amphiphilic lipid, and in particular its hydrophilic part, carries a detectable moiety, targeting moiety or a linker moiety.

Suitable detectable moieties include, but are not limited to, fluorescent moieties and moieties which can be detected by an enzymatic reaction or by specific binding of a detectable molecule (e.g. a fluorescence-labelled antibody), fluorescent moieties (such as, for example, fluorescein or rhodamine B) being preferred. According to a particular embodiment, the nanoparticle of the invention comprises one or more than one phospholipid (e.g. phosphatidylethanolamine) carrying a fluorescent moiety. Typically, the amount of amphiphilic lipid(s) comprising a detectable moiety, and in particular a fluorescent moiety, is in the range of 0.01-2 wt-%, in particular 0.1-1.5 wt-%, and preferably 0.5-1 wt-%, relative to the total weight of the polymer(s) (1) and cargo compound(s) (2) of the nanoparticle.

Targeting moieties are capable of binding specifically to a target molecule (e.g. a cell surface molecule characteristic for a particular type of cells), which allows nanoparticles comprising amphiphilic lipids with such target moieties to accumulate at a particular target site (e.g. in a particular organ or tissue) within a subject's body. Suitable targeting moieties include, but are not limited to, antibodies (such as conventional and single-domain antibodies), antigen-binding fragments and derivatives thereof, as well as ligands and ligand analogues of cell surface receptors. Typically, the amount of amphiphilic lipid(s) comprising a targeting moiety, and in particular an antibody or antigen-binding fragment thereof, is in the range of 0.01-10 wt-%, in particular 0.1-7 wt-%, and preferably 0.5-5 wt-%, relative to the total weight of the polymer(s) (1) and cargo compound(s) (2) of the nanoparticle.

Linker moieties allow for the attachment of, for example, targeting and/or labelling compounds to the amphiphilic lipid, in particular via covalent coupling so as to form amphiphilic lipids comprising detectable or targeting moieties as described herein. Thus, compounds such as targeting or labeling compounds can be attached (e.g. coupled covalently) to the surface of nanoparticles comprising (incorporated in their polymeric shell) one or more than one amphiphilic lipid carrying a linker moiety. Suitable linker moieties have a reactive function, such as a maleimide, carboxy, succinyl, azido, 2-pyridyldithio, 2,4-dichlorotriazinyl, sulfhydryl, amino, biotinyl or aldehyde group, with maleimide being preferred. Typically, the amount of amphiphilic lipid(s) comprising a linker moiety is in the range of 0.01-10 wt-%, in particular 0.1-7 wt-%, and preferably 0.5-5 wt-%, relative to the total weight of the polymer(s) (1) and cargo compound(s) (2) of the nanoparticle.

Further suitable agents which can be coupled to the amphiphilic lipid used in nanoparticles of the invention (as described for detectable and targeting compounds herein) include compounds which are capable of making the nanoparticles invisible to the immune system (such as folic acid), increase the circulation time of the nanoparticles within the subject and/or slow down elimination of the nanoparticles.

The nanoparticle of the invention may comprise more than one type of amphiphilic lipid described herein, thus combining different functions such as targeting and labeling on one and the same nanoparticle.

The components of the nanoparticles of the invention as well as the ingredients of compositions of such nanoparticles, in particular the carrier, are, expediently, pharmaceutically acceptable. This does not necessarily apply to the cargo compounds (2), where (cyto-)toxicity may be a feature of its pharmaceutical activity.

The term "pharmaceutically acceptable", as used herein, refers to a compound or material that does not cause acute toxicity when nanoparticles of the invention or a composition thereof is administered in the amount required for medical treatment or prophylaxis.

Polymers having a defined degree of polymerization such as those comprised by the nanoparticles of the present invention can be prepared by a method comprising:
(i) providing a liquid phase comprising monomer selected from C₁-C₁₀-alkyl cyanoacrylates and C₁-C₆-alkoxy-C₁-C₁₀-alkyl cyanoacrylates dissolved in an aprotic water-miscible organic solvent or a mixture of two or more aprotic water-miscible organic solvents, where the liquid phase may moreover contain one or more than one C₁-C₃-alkanol;
(ii) adding one or more than one base selected from metal hydroxides and metal alkoxides to the liquid phase, where, in case that the base is selected from metal hydroxides and the liquid phase provided in (i) does not contain any C₁-C₃-alkanols, the metal hydroxide(s) is/are added in the presence of one or more than one C₁-C₃-alkanol;
(iii) allowing an equilibrium of depolymerization and repolymerization processes to establish such that the polymer having the defined degree of polymerization is formed; and
(iv) quenching the polymerization reaction.

Examples of the suitable monomers in step (i) of the method include, but are not limited to, methyl 2-cyanoacrylate, 2-methoxyethyl 2-cyanoacrylate, ethyl 2-cyanoacrylate, n-butyl 2-cyanoacrylate, isobutyl 2-cyanoacrylate, *sec*-butyl 2-cyanoacrylate, tert-butyl 2-cyanoacrylate, n-hexyl-2-cyanoacrylate, isohexyl-cyanoacrylate, and n-octyl 2-cyanoacrylate, wherein butyl 2-cyanoacrylates (in particular n-butyl-2-cyanoacrylate), ethyl-2-cyanoacrylate and n-octylcyanoacrylate are preferred, and butyl 2-cyanoacrylate such as n-butyl-2-cyanoacrylate is particularly preferred.

According to particularly preferred embodiments, there is only one type of monomer liquid phase of step (i) of the method that is selected from C₁-C₁₀-alkyl cyanoacrylates and C₁-C₆-alkoxy-C₁-C₁₀-alkyl cyanoacrylates as described herein.

According to the present invention, the base, which is added in step (ii) of the method for preparing a polymer having a defined degree of polymerization, is selected from metal hydroxides and metal alkoxides.

Suitable metal hydroxides include, but are not limited to, alkali metal hydroxides and alkaline earth metal hydroxides, and are preferably alkali metal hydroxides.

More preferably, the metal hydroxides are selected from lithium hydroxide, sodium hydroxide, potassium hydroxide, and mixtures thereof.

Even more preferably, the metal hydroxides are selected from potassium hydroxide and sodium hydroxide. In particular, the metal hydroxide is sodium hydroxide.

Preferably, the C₁-C₃-alkanol is selected from methanol, ethanol, isopropanol and mixtures thereof.

More preferably, the C₁-C₃-alkanol is selected from methanol, ethanol and mixtures thereof, and is in particular methanol.

Suitable metal alkoxides include, but are not limited to, alkali metal alkoxides and alkaline earth metal alkoxides, preferably alkali metal C₁-C₄-alkoxides and alkaline earth metal C₁-C₄-alkoxides.

C₁-C₄-alkoxides are for example methoxide, ethoxide, propoxide, isopropoxide, butoxide, sec-butoxide, isobutoxide and tert-butoxide.

More preferably, the metal alkoxides are selected from alkali metal C₁-C₄-alkoxides.

In particular, the metal alkoxides are selected from sodium C₁-C₄-alkoxides and potassium C₁-C₄-alkoxides, for example sodium methoxide, sodium ethoxide, sodium propoxide, potassium tert-butoxide and mixtures thereof, wherein sodium methoxide is particularly preferred.

If a C₁-C₃-alkanol is present in the liquid phase provided in step (i), this is present in such an amount that the molar ratio of alkanol to base added in step (ii) is at least 1:1, e.g. 1:1 to 100:1, in particular 1:1 to 10:1.

If a C₁-C₃-alkanol is present in the liquid phase provided in step (i), the weight ratio of C₁-C₃-alkanol to aprotic water-miscible organic solvent is from 1:1 to 1:100, preferably 1:10 to 1:100.

In case that the base is a metal hydroxide and the liquid phase of step (i) does not contain any C₁-C₃-alkanol, the metal hydroxide is mandatorily added together with a C₁-C₃-alkanol.

Accordingly, in case that the liquid phase of step (i) does not contain any C₁-C₃-alkanol, the base which is added in step (ii) of the method according to the present invention is selected from
- mixtures of one or more than one metal hydroxide with one or more than one C₁-C₃-alkanol, as defined above, optionally in the form of aqueous solutions, and
- one or more than one metal alkoxide, optionally in the form of aqueous solutions.

Typically, the molar ratio of the metal hydroxide(s) to the C₁-C₃-alkanol(s), which may be added as base in step (ii) of the method according to the present invention, is in the range of from 5:1 to 1:100, preferably in the range of from 1:1 to 1:100, in particular in the range of from 1:1 to 1:10.

In a preferred embodiment, the base, which is added in step (ii) of the method according to the present invention, is selected from
- mixtures of one or more than one alkali metal hydroxide with one or more than one C₁-C₃-alkanol, selected from methanol, ethanol and isopropanol, optionally in the form of aqueous solutions, and
- one or more than one alkali metal alkoxide, optionally in the form of aqueous solutions.

In an even more preferred embodiment, the base, which is added in step (ii) of the method according to the present invention, is selected from
- mixtures of one or more than one alkali metal hydroxide with one or more than one C₁-C₃-alkanol, selected from methanol, ethanol and isopropanol, in the form of aqueous solutions and
- one or more than one alkali metal alkoxide, in the form of aqueous solutions.

Specifically, the base, which is added in step (ii) of the method according to the present invention, is selected from
- a mixture of sodium hydroxide with methanol in the form of an aqueous solution and
- sodium methoxide in the form of an aqueous solution.

If the metal hydroxide and/or the metal alkoxide is added in step (ii) of the method according to the present invention in the form of an aqueous solution, the concentration of the metal hydroxide and/or the metal alkoxide in the aqueous solution is typically in the range of from 1 mM to 10000 mM, preferably in the range of 5 mM to 5000 mM.

In a particularly preferred embodiment, the polymers having a defined degree of polymerization such as those comprised by the nanoparticles of the present invention can be prepared by a method comprising:
(i) providing a liquid phase comprising monomer selected from C₁-C₁₀-alkyl cyanoacrylates and C₁-C₆-alkoxy-C₁-C₁₀-alkyl cyanoacrylates dissolved in an aprotic water-miscible organic solvent or a mixture of two or more aprotic water-miscible solvents;
(ii) adding one or more than one alkali metal alkoxide to the liquid phase;
(iii) allowing an equilibrium of depolymerization and repolymerization processes to establish such that the polymer having the defined degree of polymerization is formed; and
(iv) quenching the polymerization reaction.

Suitable and preferable alkali metal alkoxides of this embodiment are as defined above.

It is further preferred that the alkali metal alkoxide(s), as defined above, is/are added in form of an aqueous solution to the liquid phase.

Without wishing to be bound by theory, it is assumed that the initially formed polymer having a very high degree of polymerization (and thus very high molecular mass) ("parent" polymer) undergoes depolymerization by unzipping (i.e. release of monomeric units) from the termini. Said depolymerization is accompanied by a simultaneous repolymerization of the unzipped monomer (i.e. optionally alkoxylated alkylcyanoacrylate) that forms polymers having a lower degree of polymerization (and thus lower molecular mass) ("daughter" polymer). It is assumed that the equilibrium of the depolymerization-repolymerization reaction in aprotic water-miscible organic solvent(s) is affected by the relative amounts of free monomer and the added base, as defined above, and the polarity of the solvent(s), and that thus the degree of polymerization (and thus the molecular mass) of the resulting (daughter) polymer in step (iii) of the method of the present invention can be controlled by the relative amount of the base, as defined above, added in step (ii). Due to said control, polymer having a relatively high uniformity with respect to size, i.e. a dispersity Ð_{M} close to 1.0, can be obtained.

Typically, the molar ratio between the base, in particular the alkali metal alkoxide(s), added in step (ii) and the monomer of step (i) is in the range of from 1:10 to 1:3,000, in particular in the range of from 1:10 to 1:1,000, preferably in the range of from 1:10 to 1:100 and especially in the range of from 1:15 to 1:70.

The liquid phase of step (i), in particular, is a phase that is liquid at 25°C and a pressure of 100 kPa.

Suitable aprotic water-miscible organic solvents for use in the liquid phase of step (i) include, but are not limited to, tetrahydrofuran, dioxane (in particular 1,4-dioxane), dimethoxyethane (in particular 1,2-dimethoxyethane) and mixtures thereof, wherein tetrahydrofuran is particularly preferred.

The liquid phase of step (i) can be essentially free of water. The liquid phase of step (i) can (also) be essentially free of organic solvents other than aprotic water-miscible solvents, or essentially free of organic solvents other than tetrahydrofuran, dioxane and dimethoxyethane.

With reference to the liquid phase of step (i), the term "essentially free of" [water or other organic solvent] refers to amounts of less than 10 vol-%, preferably less than 5 vol-%, and especially less than 3 vol-% [water or other organic solvent] relative to the volume of the liquid phase.

The solvent(s) of step (i) can be spiked with one or more than one acid having a pkₐ < 1. The term "pkₐ" is used herein as an abbreviation of the negative decimal logarithm of the acid dissociation constant as generally understood in the art (cf., e.g., Suggs: Organic Chemistry, Barron's Educational Series, 2002). The amount of said acid(s) is expediently chosen such that the polymerization of the monomer in the liquid phase of step (i) (i.e. in the absence of the base) is significantly slowed or prevented. Such amount is referred to herein as "stabilizing amount". If the solvent(s) in step (i) is/are spiked with acid(s) having a pkₐ < 1, the molar ration between the acid equivalents of said acid(s) and the monomer of step (i) is in the range of from 1:100 to 1:1,000, in particular from 1:150 to 1:500.

Suitable acids for spiking the solvent(s) of step (i) include, but are not limited to, trifluoroacetic acid, trichloroacetic acid, hydrochloric acid, nitric acid, picric acid, sulfuric acid, methanesulphonic acid, trifluoromethanesulphonic acid, p-toluenesolfonic acid and mixtures thereof, wherein trifluoroacetic acid is particularly preferred.

The quenching (stopping) of the (re-)polymerization reaction in the liquid phase in step (iv) may involve mixing the liquid phase obtained in step (iii) (that contains the polymer having the defined degree of polymerization) with an aqueous acid solution so as to precipitate the polymer. Preferably, the aqueous acid solution used for polymer precipitation has a pH of 1.2 or less, in particular 1.0 or less, for example, a pH in the range of from 0.8-1.2 or from 0.9-1.1, and is used in an excess of at least 8 volumes, such as from 8 to 10 volumes, aqueous acid solution per 1 volume liquid phase obtained in step (iii). Suitable aqueous acid solutions for polymer precipitation include, but are not limited to aqueous solutions of hydrochloric acid, phosphoric acid, nitric acid, sulfuric acid, trifluoroacetic acid, and mixtures thereof, wherein hydrochloric acid is particularly preferred.

The method for preparing polymer (1) according to the present invention can further comprise purification steps. Suitable methods for purifying a polymer (such as the polymer produced by the method of the present invention) from a liquid phase are known in the art. For example, the above-mentioned polymer precipitation with an aqueous acid solution represents a purification step. Further examples of purification steps useful in the method of the present invention for purifying the resulting polymer include, but are not limited to, lyophilization (freeze-drying) (e.g. of polymer precipitated as described above), filtration steps, partial or complete removal of the solvents in the liquid phase obtained in step (iii), and partial or complete exchange of the solvents in the liquid phase obtained in step (iii).

A further embodiment of the present invention relates to nanoparticles, as defined above, where the C-H acidic end(s) of the polymer(s) is/are functionalized with a linear or branched C₁-C₁₀-alkyl radical or a linear or branched C₂-C₁₀-alkenyl radical comprising at least one double bond of which at least one is terminal, where the C₁-C₁₀-alkyl radical and the C₂-C₁₀-alkenyl radical independently of each other are unsubstituted or substituted by one or more, e.g. 1, 2 or 3, preferably 1 or 2, substituent(s) selected from, C₁-C₁₀-alkoxy, C₁-C₁₀-alkylcarbonyl, C₁-C₁₀-alkoxycarbonyl and nitro.

The term "C-H acidic end", as used herein, refers to the terminus of the carbon-backbone of polymer(s) (1), which carries the tertiary C-H group, as illustrated by the general formula (II) 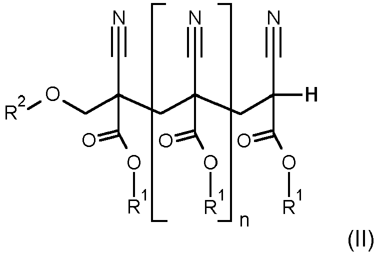 wherein
- n: is a whole number from 58 to 328,
- R¹: is C₁-C₁₀-alkyl or C₁-C₆-alkoxy-C₁-C₁₀-alkyl,
- R²: is hydrogen or C₁-C₄-alkyl and
- H: indicates the tertiary C-H acidic proton.

This tertiary C-H group is slightly acidic due to the electron withdrawing effect of the alkoxycarbonyl and nitrilegroup that are attached to it.

The terms "functionalized", "capped" and "end-capped", are used synonymously herein and do all refer to the same modification (functionalization) of the C-H acidic end of the polymer(s) (1), as described above. The functionalization of the C-H acidic end (end capping reaction mechanism) of polymer(s) (1) is depicted in scheme 1 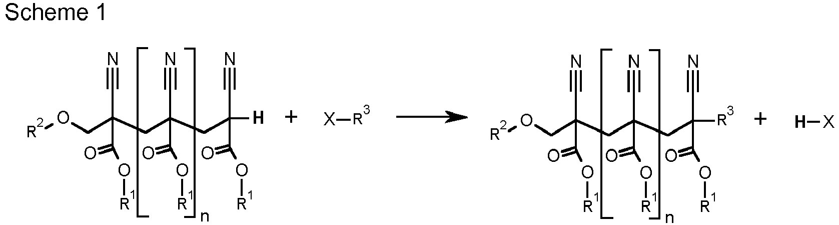 wherein
- n: is a number from 58 to 328,
- R¹: is C₁-C₁₀-alkyl or C₁-C₆-alkoxy-C₁-C₁₀-alkyl,
- R²: is hydrogen or C₁-C₄-alkyl,
- R³: is a linear or branched C₁-C₁₀-alkyl radical or a linear or branched C₂-C₁₀-alkenyl radical comprising at least one double bond of which at least one is terminal, where the C₁-C₁₀-alkyl radical and the C₂-C₁₀-alkenyl radical independently of each other are unsubstituted or substituted by one or more substituent(s) selected from C₁-C₁₀-alkoxy, C₁-C₁₀-alkylcarbonyl, C₁-C₁₀-alkoxycarbonyl and nitro,
- H: indicates the tertiary C-H acidic proton and
- X: is a leaving group, such as a halogen or a sulfonate, e.g. triflate, tosylate and the like.

The functionalization (end-capping) of the optionally alkoxylated poly(alkyl cyanoacrylates) of the present invention (polymer(s) (1)) leads to functionalized (end-capped or terminated) poly-(alkyl/alkoxyalkyl)-cyanoacrylates (hereinafter also abbreviated as tPACA's) that are virtually stable against depolymerization processes. Furthermore, the introduction of a terminal double bond at the terminus of the polymer(s) (1) enables further modification reactions, such as cross-linking reactions or the covalent attachment of other molecules, e.g. a pharmaceutically active compound (drug substance), before or after the preparation of the nanoparticles.

In a preferred embodiment of the present invention, the C-H acidic end(s) of the polymer(s) comprised in the nanoparticles, as defined above, is/are functionalized with a linear or branched C₁-C₆-alkyl radical or a linear or branched C₂-C₁₀-alkenyl radical comprising a terminal double bond, where the C₁-C₆-alkyl radical and the C₂-C₁₀-alkenyl radical independently of each other are unsubstituted or substituted with C₁-C₁₀-alkoxycarbonyl.

In a more preferred embodiment of the present invention, the C-H acidic end(s) of the polymer(s) comprised in the nanoparticles, as defined above, is/are functionalized with a linear or branched C₂-C₁₀-alkenyl radical comprising a terminal double bond, where the C₂-C₁₀-alkenyl radical is unsubstituted or substituted with C₁-C₁₀-alkoxycarbonyl.

In an even more preferred embodiment of the present invention, the C-H acidic end(s) of the polymer(s) comprised in the nanoparticles, as defined above, is/are functionalized with a linear or branched C₂-C₆-alkenyl radical comprising a terminal double bond, where the C₂-C₆-alkenyl radical is unsubstituted or substituted with C₁-C₁₀-alkoxycarbonyl.

In still more preferred embodiment of the present invention, the C-H acidic end(s) of the polymer(s) comprised in the nanoparticles, as defined above, is/are functionalized with a linear or branched C₂-C₆-alkenyl radical comprising a terminal double bond, where the C₂-C₆-alkenyl radical is substituted with C₁-C₁₀-alkoxycarbonyl.

In a particularly preferred embodiment of the present invention, the C-H acidic end(s) of the polymer(s) comprised in the nanoparticles, as defined above, is/are functionalized with 2-(C₁-C₁₀-alkoxycarbonyl)allyl, in particular with 2-(C₁-C₅-alkoxycarbonyl)allyl, especially with 2-(butoxycarbonyl)allyl.

The functionalized (capped) polymers comprised in the nanoparticles of the present invention can be prepared by a method as defined above for non-functionalized (non-capped) polymers, wherein the liquid phase obtained in step (iii) containing the polymer having the defined degree of polymerization is reacted with a terminator compound prior to the quenching in step (iv).

The point of time of the addition of the terminator compound is typically at the end of step (iii), where the depolymerization and repolymerization process is in equilibrium, i.e. the polymer having a defined degree of polymerization has formed.

The terminator compound is a compound of the general formula X-R³, as depicted in Scheme 1, wherein X is a leaving group and R³ is a linear or branched C₁-C₁₀-alkyl radical or a linear or branched C₂-C₁₀-alkenyl radical comprising at least one double bond of which at least one is terminal, where the C₁-C₁₀-alkyl radical and the C₂-C₁₀-alkenyl radical independently of each other are unsubstituted or substituted by one or more, e.g. 1, 2 or 3, preferably 1 or 2, substituent(s) selected from C₁-C₁₀-alkoxy, C₁-C₁₀-alkylcarbonyl, C₁-C₁₀-alkoxycarbonyl and nitro.

Typically, the leaving group X is selected from leaving groups that are commonly used in nucleophilic substitution reactions, e.g. halogens or sulfonates, such as triflate, tosylate and the like.

Preferably, the leaving group X is selected from halogens, such as fluorine, chlorine, bromine and iodine. More preferably the leaving group X is selected from chlorine and bromine.

In particular the leaving group X is bromine.

Preferably, the terminator compound is selected from compounds X-R³, wherein X is a halogen and R³ is a linear or branched C₁-C₆-alkyl radical or a linear or branched C₂-C₁₀-alkenyl radical comprising a terminal double bond, where the C₁-C₆-alkyl radical and the C₂-C₁₀-alkenyl radical independently of each other are unsubstituted or substituted with C₁-C₁₀-alkoxycarbonyl.

More preferably, the terminator compound is selected from compounds X-R³, wherein X is a halogen and R³ is a C₂-C₁₀-alkenyl radical comprising a terminal double bond, where the C₂-C₁₀-alkenyl radical is unsubstituted or substituted with C₁-C₁₀-alkoxycarbonyl.

Even more preferably, the terminator compound is selected from compounds X-R³, wherein X is a halogen and R³ is a C₂-C₆-alkenyl radical comprising a terminal double bond, where the C₂-C₆-alkenyl radical is unsubstituted or substituted with C₁-C₁₀-alkoxycarbonyl.

Still more preferably, the terminator compound is selected from compounds X-R³, wherein X is a halogen and R³ is a linear or branched C₂-C₆-alkenyl radical comprising a terminal double bond, where the C₂-C₆-alkenyl radical is substituted with C₁-C₁₀-alkoxycarbonyl.

It is further preferred that the double bond or the terminal double bond in C₂-C₁₀-alkenyl and C₂-C₆-alkenyl is not located on the carbon atom which carries the leaving group X.

Particularly, the terminator compound is selected from C₁-C₁₀-alkyl 2-chloromethyl acrylate and C₁-C₁₀-alkyl 2-bromomethyl acrylate.

More particularly, the terminator compound is selected from C₁-C₁₀-alkyl 2-bromomethyl acrylate.

Especially, the terminator compound is ethyl 2-(bromomethyl) acrylate (EBMA).

The reaction of the polymer with the terminator compound can be performed according to procedures that are known in the art, such as for example the process described by Kohsaka et al. (Macromol Chem Phys 216(14):1534-1539, 2015).

Typically, the molar ratio of the terminator compound to the polymer obtained in step (iii) having the defined degree of polymerization is in the range of 1:3 to 30:1, preferably in the range of 1:2 to 1:25, in particular in the range of 1:1 to 1:20.

The invention further provides a method for preparing nanoparticles as well as nanoparticles obtainable by said method. The method of the invention for preparing nanoparticles comprises:
(a) providing a hydrophobic liquid phase comprising:
   (1) one or more than one polymer:
      - comprising from 60 to 330 monomeric constituents selected from C₁-C₁₀-alkyl cyanoacrylates and C₁-C₆-alkoxy-C₁-C₁₀-alkyl cyanoacrylates, and
      - having a dispersity (Ð_{M}) of less than 3.0, and
   (2) one or more than one cargo compound selected from pharmaceutically active agents, cosmetically active agents and nutritional supplements
      dissolved in a water-immiscible organic solvent or a mixture of two or more water-immiscible organic solvents;
(b) providing a hydrophilic liquid phase;
(c) finely dispersing the hydrophobic liquid phase in the hydrophilic liquid phase so as to form an emulsion; and
(d) removing at least part of the organic solvent(s) from the emulsion so as to obtain a suspension of nanoparticles in the hydrophilic solvent.

In contrast to methods such as interfacial polymerization or emulsion polymerization, the method of the invention starts with preformed polymer which allows for a better control of the properties of the polymer, and thus of the resulting nanoparticles, as well as for a reduction of the residual monomer content. Furthermore, a separate polymer manufacturing process allows a close control and detailed characterization of the polymer as a pharmaceutical ingredient. Such an accurately defined pre-manufactured polymer can be advantageously used as an ingredient of nanoparticles for pharmaceutical applications where a precise definition of the medicament and its ingredients is particularly crucial.

The polymer(s) (1) and the cargo compound(s) (2) used in the method of the invention are preferably those defined above for the nanoparticles of the invention. According to particularly preferred embodiments, only one type of poly(C₁-C₁₀-alkyl cyanoacrylate) or poly(C₁-C₆-alkoxy-C₁-C₁₀-alkyl cyanoacrylate) as described herein is used for preparing nanoparticles as described herein, i.e. the hydrophobic liquid phase in step (a) comprises only one type of poly(C₁-C₁₀-alkyl cyanoacrylate) or poly(C₁-C₆-alkoxy-C₁-C₁₀-alkyl cyanoacrylate).

The terms "hydrophobic" and "hydrophilic" are used here as relative terms; i.e. the hydrophilic liquid phase provided in (b) is more polar than the hydrophobic liquid phase provided in (a); the polarity difference being of course not marginal, but being so great that mixing the two phases does not provide a homogenous solution.

The organic solvents useful for providing the hydrophobic liquid phase in step (a) of the method of the invention are water-immiscible solvents. The term "water-immiscible organic solvents", as used herein, refers to organic solvents having a solubility in water of less than about 10 wt-%, in particular less than about 5 wt-%, and preferably less than about 3 wt-%. Water-immiscible organic solvents for use in step (a) are preferably volatile, i.e. are liquid at room temperature (25°C) and have a boiling point of 150°C or less at standard pressure (100 kPa). Examples of suitable water-immiscible organic solvents include, but are not limited to, chloroform, methylene chloride, trichloroethylene, trichloro-trifluoroethylene, tetrachloroethane, trichloroethane, dichloroethane, dibromoethane, ethyl acetate, phenol, toluene, xylene, ethyl-benzene, benzyl alcohol, creosol, methyl-ethyl ketone, methyl-isobutyl ketone, hexane, heptane, furan and non-cyclic aliphatic ethers such diethyl ether, as well as mixtures thereof, chloroform being preferred.

The hydrophilic solvent used for providing the hydrophilic liquid phase of step (b) of the method of the invention is preferably water.

Emulsion solvent evaporation methods, wherein the volume of the hydrophilic phase is very high relative to the volume of the hydrophobic phase, yield very dilute nanoparticle suspensions, which may require processing steps to increase the concentration of nanoparticles in the suspension to a concentration sufficiently high for the ultimate use. The volume ratio of hydrophobic liquid phase : hydrophilic liquid phase is generally in the range of from 1:100 to 1:1, preferably in the range of from 1:9 to 1:1.5 or about 1:2.

The hydrophobic liquid phase is finely dispersed in the hydrophilic liquid phase so as to form an emulsion of fine droplets of the hydrophobic liquid distributed throughout the hydrophilic liquid. This emulsion may be obtained by applying shear forces, for example by thorough mixing using a static mixer, by ultrasound, by homogenization under pressure, e.g. under a pressure of at least 5,000 kPa, such as from 20,000 to 200,000 kPa, preferably from 50,000 to 100,000 kPa, or by combining any of these homogenization methods. The emulsion of the hydrophobic liquid in the hydrophilic liquid can be prepared in a two-step process, wherein the two phases are first mixed, e.g. with a static mixer (rotator/stator-type mixer), so as to obtain a pre-emulsion which, in a second step, is further homogenized ultrasonically and/or using a high pressure homogenizer so as to reduce the size of the hydrophobic liquid droplets. The shear forces may be applied for a time of from 1-12 min, in particular from 4-10 min. For example, ultrasound may be applied for 1-10 min, in particular from 3-7 min, with amplitude in the range of from 50-100%, in particular 60-100%.

At least part of the organic solvent(s) is then removed from the homogenized mixture so as to obtain a suspension of nanoparticles in a hydrophilic, preferably aqueous, medium (comprising the hydrophilic solvent). Suitable measures for removing organic solvent from a homogenized mixture, such as in step (d) of the method of the invention, are known in the art and include, but are not limited to, evaporation, extraction, diafiltration, pervaporation, vapor permeation and filtration. The concentration of organic solvent in the hydrophilic suspension medium of the nanoparticles is expediently reduced to below the solubility of the organic solvent in the said medium, in particular to a concentration of less than about 5 wt-%, less than about 3 wt-%, less than about 1 wt-% and preferably less than about 0.1 wt-%. Preferably, the organic solvent(s) is/are removed to an extent that the resulting suspension of nanoparticles is pharmaceutically acceptable or acceptable according to the ICH (International Committee on Harmonization) guidelines, respectively.

Optionally, the method of the invention may further comprise purification steps such as a removal of precipitates and agglomerates of the cargo compound(s) (2), e.g. by filtration, and/or a partial or complete exchange of the suspension medium, e.g. by dialysis.

The method of the invention can yield preparations of nanoparticles having a relatively high uniformity with respect to size, for example preparations wherein the majority of the nanoparticles has a diameter of less than 500 nm, less than 300 nm and in particular less than 200 nm, such as in the range of from 1-500 nm, 10-300 nm, preferably in the range of from 50-200 nm, and more preferably in the range of from 90-130 nm. In particular, nanoparticle preparations obtained with the method of the invention can have PDI values as determined, for example, by dynamic light scattering (DLS) as described herein of 0.5 or less, 0.3 or less, in particular 0.2 or less. Nonetheless, the nanoparticle preparation may be processed further (e.g. by filtration) to remove nanoparticles having diameters outside a desired range.

The hydrophilic liquid phase of step (b) can comprise one or more than one nanoparticle-stabilizing agent as described above dissolved in the hydrophilic solvent(s). The presence of a nanoparticle-stabilizing agent allows for the formation of highly stable nanoparticles, high encapsulation efficiency as well as high absolute drug loading.

The term "encapsulation efficiency" (EE) refers to the amount of cargo compound(s) encapsulated in nanoparticles relative to the total amount of cargo compound(s) used for preparing the nanoparticles. The method of the present invention allows for encapsulation efficiencies of at least 40%, at least 50%, at least 60%, at least 70% or even at least 80%, cargo compound(s) relative to the total weight of the polymer(s) (1) and cargo compound(s) (2) used in the preparation of the nanoparticle.

The term "absolute drug loading" (AL) refers to the weight of cargo compound(s) encapsulated in the nanoparticles relative to the total weight of the polymer(s) (1) and cargo compound(s) in the nanoparticles. Absolute drug loading is one of the most important measures considering the application dose. In contrast to previously described nanoparticles based on poly(alkyl cyanoacrylates), the nanoparticles according to the present invention can have significantly increased absolute drug loadings such as at least 5 wt-%, at least 10 wt-%, least 20 wt-%, at least 40 wt-%, at least 50 wt-%, at least 60 wt-%, or even at least 70 wt-%.

The concentration of nanoparticle-stabilizing agent(s) in the hydrophilic phase provided in step (b) is typically in the range of from 50% to 150%, in particular from 80% to 120% and preferably from 90% to 110%, of its critical micelle concentration, for example in the range of from 5 mM to 15 mM, in particular from 8 mM to 12 mM and specifically from 9 mM to 11 mM.

The term "critical micelle concentration" (CMC) refers to the concentration of a surfactant above which micelles form.

The hydrophilic liquid phase provided in step (b) can further comprise one or more than one uptake mediator selected from polyoxyethylene sorbitan fatty acid esters as described herein. Particularly suitable concentrations of the sorbitan fatty acid ester(s) in the hydrophobic liquid phase are in the range of from 50% to 150%, in particular from 80% to 120% and preferably from 90% to 110%, of its critical micelle concentration, for example in the range of from 6 µM to 18 µM, in particular from 9.6 µM to 14.4 µM and specifically from 10.8 µM to 13.2 µM.

The hydrophilic liquid phase of step (b), in particular if the hydrophilic solvent is water, can comprise one or more than one (further) substance selected from pH buffering agents and salts.

The hydrophobic liquid phase provided in step (a) can comprise one or more than one sorbitan fatty acid ester as described herein. Particularly suitable concentrations of the sorbitan fatty acid ester(s) in the hydrophobic liquid phase are in the range of from 0.1 M to 0.2 M, specifically from 0.12 M to 0.18 M.

The hydrophobic liquid phase provided in step (a) can further comprise one or more than one amphiphilic lipid as described herein.

The invention further provides a method for preparing nanoparticles from polymers whose C-H acidic end(s) are functionalized (capped), the method comprising the method as defined above for the preparation of nanoparticles from non-functionalized (non-capped) polymers, wherein the C-H acidic end(s) of the polymer(s) in the hydrophobic liquid phase of step (a) is/are functionalized with a linear or branched C₁-C₁₀-alkyl radical or a linear or branched C₂-C₁₀-alkenyl radical comprising at least one double bond of which at least one is terminal, where the C₁-C₁₀-alkyl radical and the C₂-C₁₀-alkenyl radical independently of each other are unsubstituted or substituted by one or more substituent(s) selected from C₁-C₁₀-alkoxy, C₁-C₁₀-alkylcarbonyl, C₁-C₁₀-alkoxycarbonyl and nitro.

Regarding preferred and particularly preferred functionalized polymers that are applied in the hydrophobic liquid phase of step (a), reference is made to the statements given above in connection with the nanoparticles based on functionalized (capped) polymers.

The nanoparticles of the invention can be used in methods for treating a disorder by therapy and/or for prophylaxis against a disorder. Said methods comprise administering the nanoparticles or a composition thereof to a subject (such as a human) in need of such therapy and/or prophylaxis. Expediently, an effective amount of the nanoparticles or the composition thereof is administered to the subject.

The term " effective amount", as used herein, refers to the amount of a therapy which is sufficient to reduce or ameliorate the severity and/or duration of a disorder or one or more symptoms thereof, prevent the advancement of a disorder, cause regression of a disorder, prevent the recurrence, development, onset or progression of one or more symptoms associated with a disorder, detect a disorder, or enhance or improve the prophylactic or therapeutic effect(s) of another therapy (e.g., prophylactic or therapeutic agent).

The nanoparticles of the invention can increase the bioavailability and efficacy of the encapsulated cargo compound(s) (2) by protecting said compound(s) from premature degradation in the gastrointestinal tract and/or the blood, and allowing for a sustained release thereof and/or a targeting to specific areas within the body. Following oral administration, the nanoparticles of the invention may traverse the intestinal wall and even barriers such as the blood-brain barrier.

The nanoparticles of the present invention are particularly useful in the treatment of inflammatory bowel diseases such as, for example, Crohn's disease or colitis ulcerosa. They can deliver drugs specifically to the inflamed intestinal regions. Moreover, the nanoparticles can accumulate in flamed intestinal tissue by specific passive accumulation that occurs, *inter alia,* due to negative particle surface charge and reduced particles size (cf. Collnot et al., J Control Release 161:235-246, 2012). The accumulation of the nanoparticles and prolonged drug release from the nanoparticles are particularly desirable effects for indications such as inflammatory bowel disease since they increase the local drug concentration and decrease the systemic drug availability, and thus undesirable side effects at off-target sites.

In particular embodiments, the nanoparticles of the invention used for pharmaceutical applications comprise glucocorticoid steroids as cargo compound (2). Glucocorticoids can be used in the treatment of a variety of disorders including inflammatory bowel diseases. Accordingly, nanoparticles of the present invention, wherein the cargo compound (2) is a glucocorticoid steroid as described herein, are particularly useful in the treatment of inflammatory bowel diseases as described herein.

Glucocorticoid steroids suitable for incorporation into nanoparticles of the invention for use in the treatment of inflammatory bowel diseases include, but are not limited to, budesonide, cortisone, hydrocortisone, prednisone, prednisolone, 6-alpha-methylprednisolone, dexamethasone, prednisone-21-phosphate, betamethasone-17-valerate, prednisone-21-metasulfabenzoate, tixocortol pivalate, beclomethasone dipropionate and fluticasone, with budesonide being particularly preferred.

Glucocorticoid steroids influence a wide range of physiological processes within a subject's body. For treatment of local symptoms, such as inflammations in intestinal regions of patients suffering from inflammatory bowel diseases, with systemically effective glucocorticoid steroids, such as hydrocortisone, prednisone, prednisolone, 6-alpha-methylprednisolone, cortisone and dexamethasone, a local administration which provides a high local drug concentration but limits the systemic drug availability (and thus unwanted side effects) is particularly desirable. For this purpose nanoparticles of the present invention loaded with a glucocorticoid steroid as described herein can be used, e.g. administered orally to patients suffering from inflammatory bowel diseases.

The nanoparticles of the present invention can be provided in the form of a pharmaceutical composition comprising a plurality of nanoparticles as described herein, and a pharmaceutically acceptable carrier. The carrier is chosen to be suitable for the intended way of administration which can be, for example, peroral or parenteral administration, e.g. intravascular, subcutaneous or, most commonly, intravenous injection, transdermal application, or topical applications such as onto the skin, nasal or buccal mucosa or the conjunctiva.

The nanoparticles of the invention can be provided in the form of liquid pharmaceutical compositions. These compositions typically comprise a carrier selected from aqueous solutions which may comprise one or more than one water-soluble salt and/or one or more than one water-soluble polymer. If the composition is to be administered by injection, the carrier is typically an isotonic aqueous solution (e.g. a solution containing 150 mM NaCl, 5 wt-% dextrose or both). Such carrier also typically has an appropriate (physiological) pH in the range of from about 7.3-7.4.

Alternatively, the nanoparticles of the invention can be provided in the form of solid or semisolid pharmaceutical compositions, e.g. for peroral administration or as a depot implant. Suitable carrier for these compositions include, but are not limited to, pharmaceutically acceptable polymers selected from homopolymers and copolymers of N-vinyl lactams (especially homopolymers and copolymers of N-vinyl pyrrolidone, e.g. polyvinylpyrrolidone, copolymers of N- vinyl pyrrolidone and vinyl acetate or vinyl propionate), cellulose esters and cellulose ethers (in particular methylcellulose and ethylcellulose, hydroxyalkylcelluloses, in particular hydroxypropylcellulose, hydroxyl-alkylalkylcelluloses, in particular hydroxypropylmethylcellulose, cellulose phthalates or succinates, in particular cellulose acetate phthalate and hydroxypropylmethylcellulose phthalate, hydroxypropylmethylcellulose succinate or hydroxypropylmethylcellulose acetate succinate), high molecular weight polyalkylene oxides (such as polyethylene oxide and polypropylene oxide and copolymers of ethylene oxide and propylene oxide), polyvinyl alcohol-polyethylene glycol-graft copolymers, polyacrylates and polymethacrylates (such as methacrylic acid/ethyl acrylate copolymers, methacrylic acid/methyl methacrylate copolymers, butyl methacrylate/2- dimethylaminoethyl methacrylate copolymers, poly(hydroxyalkyl acrylates), poly(hydroxyalkyl methacrylates)), polyacrylamides, vinyl acetate polymers (such as copolymers of vinyl acetate and crotonic acid, partially hydrolyzed polyvinyl acetate), polyvinyl alcohol, oligo- and polysaccharides such as carrageenans, galactomannans and xanthan gum, alginate, acacia gum, gelatin or mixtures of one or more thereof. Solid carrier ingredients may be dissolved or suspended in a liquid suspension of nanoparticles of the invention and the liquid suspension medium may be, at least partially, removed.

Freeze-dried nanoparticle preparations are particularly suitable for preparing solid or semisolid pharmaceutical compositions and dosage forms of nanoparticles of the invention. Suitable methods for freeze-drying of nanoparticles are known in the art and may include the use of cryoprotectants (e.g. trehalose, sucrose, sugar alcohols such as mannitol, surface active agents such as the polysorbates, poloxamers, glycerol and/or dimethylsulfoxide). Solid dosage forms of nanoparticles of the invention which are particularly suitable for peroral administration include, but are not limited to, capsules (e.g. hard or soft gelatin capsules), tablets, pills, powders and granules, which may optionally be coated. Coatings of peroral solid dosage forms intended for delivering the nanoparticles to particular regions within the intestine (such as to inflamed intestinal regions of patients suffering from inflammatory bowel diseases) are expediently gastro-resistant.

### Drug load and drug-polymer conjugates

As described above, it was found by the inventors that the M_{w} modifications of optionally alkoxylated poly(alkyl cyanoacrylates of the present invention (polymer(s) (1)) can improve the absolute drug load of nanoparticles, which were prepared from these polymer(s) (1). It is a sophisticated but simple approach to increase the loading capacity of polycyanoacrylate-based nanoparticles without changing the proportions of formulation components of the complex structured drug delivery system. The increase of the absolute drug loading capacity into polycyanoacrylate-based nanoparticles, in particular of poorly soluble drug substances, is of great interest for all kinds of small molecules independent of the disease to be treated.

Another method to increase the absolute drug load capacity of polycyanoacrylate-based nanoparticles is to further modify the functionalized polymer(s) (1) (tPACA) by linking a small molecule, e.g. the drug substance of interest, covalently to its terminator group, in order to obtain a tPACA-drug conjugate. This linkage is advantageously be performed by the addition of the small molecule, e.g. the particular drug substance, to the terminal double bond present on the terminator group of tPACA.

Accordingly, a further embodiment of the present invention relates to a method comprising
- the reaction of the C-H acidic end(s) of the polymer(s) (1) with a terminator compound of the general formula X-R³, wherein X is a leaving group, as defined above, and R³ is a linear or branched C₂-C₁₀-alkenyl radical comprising at least one double bond of which at least one is terminal, where the C₂-C₁₀-alkenyl radical is unsubstituted or substituted by one or more substituent(s) selected from C₁-C₁₀-alkoxy, C₁-C₁₀-alkylcarbonyl, C₁-C₁₀-alkoxycarbonyl and nitro, followed by
- the linkage of a small molecule, preferably a drug substance, to the terminal double bond of the terminator group of the thus functionalized polymer(s) (1) under suitable chemical conditions,
in order to obtain a tPACA-small molecule conjugate, preferably a tPACA-drug conjugate, where the small molecule or the drug-substance is covalently linked to the tPACA.

The covalent linkage of the small molecule, e.g. the drug substance of interest, to the terminal double bond of the terminator group of tPACA can principally be performed through any reaction known to the skilled person that allows the addition of specific functional groups to a terminal double bond.

Advantageously, this linkage can be performed by a thiol-ene reaction, also known as "thiol-ene-click reactions". In this case, the terminal double bond of the terminator group at the tPACA chain terminus represents the "clickable end", which forms the covalent bond to the sulfur atom of the thiol group present on the small molecule, e.g. the drug substance of interest. Such a "clickable end" enables postpolymerization modifications of the tPACA molecule.

These thiol-ene click reactions can be performed either via a radical reaction or via Michael-Addition. Both variants of the "thiol-ene-click reaction" are well described in the art and are known to the skilled person.

It is preferred that the thiol-ene click reaction is performed via a Michael-Addition, where the sulfur atom of the thiol-group adds to the terminal double bond of the terminator group, which is part of a Michael-acceptor-system.

Accordingly, a preferred embodiment relates to the reaction of the C-H acidic end(s) of the polymer(s) (1) with a terminator compound of the general formula X-R³, wherein X is a leaving group, as defined above, and R³ is a linear or branched C₂-C₁₀-alkenyl radical comprising at least one double bond of which at least one is terminal, which is substituted by an electron withdrawing substituent, such as a C₁-C₁₀-alkylcarbonyl, C₁-C₁₀-alkoxycarbonyl or nitro group, e.g. ethyl 2-(bromomethyl) acrylate (EBMA).

The thusly substituted terminal double bond provides a clickable end at the tPACA chain terminus as, for example, described by Kohsaka et al. (Macromol Chem Phys 216(14):1534-1539, 2015). In this preferred embodiment, the terms "clickable" and "clickable end" refer to an olefinic moiety comprising a terminal double bond, where the terminal double bond is substituted by an electron withdrawing substituent, such as a carbonyl or nitro group, resulting in a Michael-acceptor-system, in which the unsubstituted carbon atom of the terminal double bond can undergo a selective and quantitative thiol-ene click reaction to form an anti-Markovnikov product as, for example, described by Kohsaka Y et al. and Lowe et al. (Polym. Chem. 2015; 6:1078-87, Polym. Chem. 2015; 6:3601-7, Polym. Chem. 2010; 1:17-36).

Accordingly, this preferred embodiment relates to a method comprising
- the reaction of the C-H acidic end(s) of the polymer(s) (1) with a terminator compound of the general formula X-R³, wherein X is a leaving group, as defined above, and R³ is a linear or branched C₂-C₁₀-alkenyl radical comprising at least one double bond of which at least one is terminal, which is substituted by an electron withdrawing substituent, such as a C₁-C₁₀-alkylcarbonyl, C₁-C₁₀-alkoxycarbonyl or nitro group, followed by
- the linkage of a molecule, preferably a drug substance, to the terminal double bond of the terminator group of the thus functionalized polymer(s) (1) under suitable chemical conditions,
in order to obtain a tPACA-small molecule conjugate, preferably a tPACA-drug conjugate, where the small molecule or the drug-substance is covalently linked to the tPACA.

The linkage of the small molecule, preferably a drug substance, to the terminal double bond of the terminator group of the functionalized polymer(s) (1) is performed under chemical conditions that are known to the skilled person or described in the art. Suitable chemical conditions are for example reaction conditions described by Kohsaka Y et al. and Lowe et al. (Polym. Chem. 2015; 6:1078-87, Polym. Chem. 2015; 6:3601-7, Polym. Chem. 2010; 1:17-36). This premanufactured tPACA-drug conjugate can then be used to manufacture nanoparticles having a drug-polymer ratio of 1 to 1. In this "drug-terminated method" concept, first the polymer chain having a terminator group with a clickable end is formed and second the polymer chain is virtually terminated with the drug molecule. An example of this concept is illustrated in Scheme 2.

Scheme 2: Schematic illustration of the "drug-terminated method" exemplified with a tPACA carrying a 2-(C₁-C₁₀-alkoxycarbonyl)allyl terminator group.

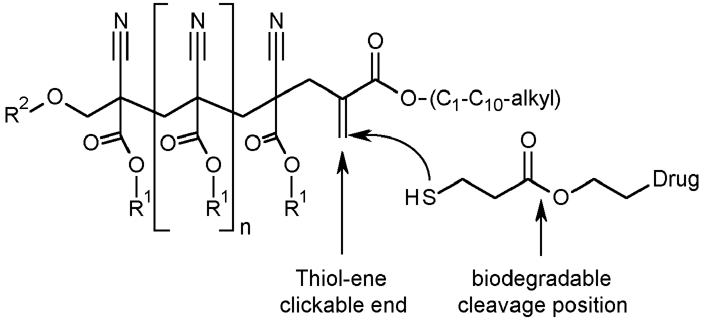

The variable n and the radicals R¹ and R² in scheme 2 are as defined above.

### EXAMPLES

### Determination of particle size and polydispersity index

In the examples described herein, size and polydispersity index (PDI) of the prepared nanoparticles were determined by cumulant analysis as defined in the international standard on dynamic light scattering (DLS) ISO13321 (1996) and ISO22412 (2008) using a Zetasizer Nano ZS device (Malvern Instruments, Germany) which yields a mean particle size (z-average diameter) and an estimate of the width of the distribution (PDI). The PDI, as indicated in the examples, is a dimensionless measure of the broadness of the size distribution which, in the Zetasizer software ranges from 0 to 1. PDI values of <0.1 indicate monodisperse samples (i.e. samples with a very uniform particle size distribution), while higher PDI values indicate more polydisperse samples.

The size of the prepared nanoparticles was also determined by nanoparticle tracking analysis (NTA) using a NanoSight LM10 device (Malvern Instruments, Germany) which yields a mean particle size as well as D10, D50 and D90 values (wherein D10, D50 and D90 designate diameters, with 10% of the particles having diameters lower than D10, 50% of the particles having diameters lower than D50, and 90% the particles having diameters lower than D90). Prior to the measurements, the nanoparticle suspensions were diluted to a concentration in the range of from 1x10⁸-5x10⁹ particles/ml.

### EXAMPLE 1 Preparation of PBCA in THF

5 ml of 0.1 mM, 1 mM or 2 mM trifluoroacetic acid (TFA) in tetrahydrofuran (THF) were mixed with n-butyl cyanoacrylate (BCA) while stirring to a final concentration of 326 mM or 653 mM BCA. 120 µl water or 120 µl of an aqueous sodium methoxide (SMeO) solution were added while stirring so as to obtain a final concentration of SMeO as indicated in Table 1 below. 100 µl-samples of the reaction mixture were taken immediately, and 1 min, 3 min, 5 min, 10 min, 30 min, 60 min, 120 min and 180 min afterwards. Stirring was continued until all samples were taken. Each sample was immediately mixed with 1 ml 0.1 M HCI in water, pH 1, so as to precipitate the PBCA formed, and kept on dry ice. The samples were lyophilized using an Alpha LSC 2-4 device (Christ) (primary drying: approximately 12 h at 37 Pa and a shelf temperature of 0°C, secondary drying: 1 h at 1 Pa and a shelf temperature 10°C). The lyophilisate was dissolved in THF to a final concentration of 5 mg/ml and the M_{W} of the PBCA in this sample was determined using an Advanced Polymer Chromatography (APC) device (Waters) (mobile phase: 100% THF, flow: 1 ml/min, 3 successively arranged columns, each packed with trimethyl silane-modified particles of ethylene-bridged hybrid material (polyethoxysilane, cf. US 6,686,035): column 1 (ACQUITY APC XT 200): 4.6 mm x 150 mm, 200 Å pore size, 2.5 µm mean particle size; each of columns 2 and 3 (ACQUITY APC XT 45): 4.6 mm x 1500mm, 45 Å pore size, 1.7 µm mean particle size) equipped with a refraction index detector and polystyrene molecular mass standards having peak molecular masses in the range of from 1,210,000 to 474 g/mol. The results are shown in Figures 1-7. The results indicate that the M_{w} of PBCA is affected by the amount of SMeO applied to the polymerization reaction. The higher the amount of SMeO, the smaller is the M_{w} obtained after 180 min polymerization time (Figures 1-7). The dependency of the M_{w} reduction of the applied amount of SMeO has been verified for all polymerization conditions given in Table 1.

**Table 1: Preparation of PBCA in THF**

| TFA [mM] | BCA [mM] | SMeO [mM] |
|---|---|---|
| 0.00 | 326 | 0.000 |
| 0.01 | 326 | 5.000 |
| 1.00 | 326 | 5.000 |
| 1.00 | 326 | 8.000 |
| 1.00 | 326 | 100.000 |
| 1.00 | 653 | 8.000 |
| 2.00 | 326 | 0.125 |
| 2.00 | 326 | 0.630 |
| 2.00 | 326 | 10.000 |
| 2.00 | 326 | 20.000 |
| 2.00 | 326 | 40.000 |
| 2.00 | 326 | 75.000 |
| 2.00 | 326 | 80.000 |

For the reaction starting from 653 mM BCA, 8 mM SMeO and 1 mM TFA in THF, the changes in M_{W} and amount of daughter PBCA and parent PBCA were determined by APC as described above (see Table 2 and Figure 8). Figure 8 illustrates the PBCA equilibration process including the complete disappearance of the parent polymer under proceeding polymerization.

**Table 2: Formation of parent PBCA and daughter PBCA**

| time [min] | parent PBCA | | daughter PBCA | |
|---|---|---|---|---|
| | Mw [g/mol] | relative amount [area-%] | Mw [g/mol] | relative amount [area-%] |
| 0 | 224,906 | 67 | 606 | 33 |
| 1 | 164,237 | 49 | 14,227 | 51 |
| 3 | 159,172 | 27 | 17,753 | 73 |
| 5 | 157,833 | 15 | 18,914 | 85 |
| 10 | 106,218 | 4 | 20,131 | 96 |
| 30 | | 0 | 21,053 | 100 |
| 60 | | 0 | 20,764 | 100 |
| 120 | | 0 | 21,388 | 100 |
| 180 | | 0 | 20,941 | 100 |

### EXAMPLE 2 Preparation of PBCA in THF -up-scale setting

5 ml of 2 mM TFA in THF were mixed with BCA while stirring to a final concentration of 326 mM BCA. Stirring was continued and 120 µl water or 120 µl of an aqueous sodium methoxide (SMeO) solution were added so as to obtain a final concentration of 0 mM, 8 mM or 80 mM SMeO. Stirring was continued for 3 h. Then, the reaction mixture was mixed with 50 ml 0.1 M HCI in water, pH 1, so as to precipitate the PBCA formed. The precipitated PBCA was separated from the liquid by filtration, washed twice with 50 ml deionized water, kept on dry ice for 30 min and then lyophilized using an Alpha LSC 2-4 device (Christ) (primary drying: approximately 12 h at 37 Pa and a shelf temperature of 0°C, secondary drying: 1 h at 1 Pa and a shelf temperature 10°C). The lyophilisate was dissolved in THF to a final concentration of 5 mg/ml and the M_{W} of the PBCA in this sample was determined by APC as described in EXAMPLE 1. For each of the conditions, the experiment was performed 3 times. The results are shown in Table 3 and Figure 9. Samples according to the claims comprise from 60 to 330 monomeric constituents selected from C₁-C₁₀-alkyl cyanoacrylates and C₁-C₆-alkoxy-C₁C₁₀-alkyl cyanoacrylates and have a dispersity of less than 3.0.

**Table 3: Up-scale preparation of PBCA in THF**

| Sample # | SMeO [mM] | M_{W} [g/mol] | dispersity (Ð_{M}) | Yield [%] |
|---|---|---|---|---|
| 2-1 | 80 | 2,160 +/- 492 | 1.57 +/- 0.15 | 88 +/- 6 |
| 2-2 | 8 | 15,223 +/- 2,178 | 1.78 +/- 0.07 | 88 +/- 6 |
| 2-3 | 0 | 210,007 +/- 36,656 | 3.617 +/- 0.8 | 91 +/- 7 |

### EXAMPLE 3 Characterization of PBCA via Thermal Gravimetric Analysis (TGA)

PBCA samples prepared as described in EXAMPLE 2 (samples 2-1, 2-2 and 2-3) were analyzed *via* TGA using a TGA 2 StarE System device (Mettler Toledo). The temperature of an about 10 mg PBCA sample was increased from room temperature to 210°C within about 18 min. The results are shown in Figure 10. The TGA results of PBCA indicated a M_{w} dependent degradation behavior. The lower the M_{w} of PBCA, the higher is the weight loss after the 18 min heating cycle.

### EXAMPLE 4 Characterization of PBCA via Differential Scanning Calorimetry (DSC)

PBCA samples prepared as described in EXAMPLE 2 (samples 2-1, 2-2 and 2-3) were analyzed *via* DSC using a DSC820 device (Mettler Toledo). An about 5 mg PBCA sample was subjected to a heating schedule as follows:
Heating schedule for sample 2-1:
   -40.0°C to 130.0°C at 10.0 K/min
   130.0°C to -40.0°C at -10.0 K/min
   -40.0°C to 130.0°C at 10.0 K/min
   130.0°C at -40.0°C at -10.0 K/min
   -40.0°C at 130.0°C at 10.0 K/min
Heating schedule for sample 2-2:
   -40.0°C to 160.0°C at 10.0 K/min
   160.0°C to -40.0°C at -10.0 K/min
   -40.0°C to 160.0°C at 10.0 K/min
   160.0°C at -40.0°C at -10.0 K/min
   -40.0°C at 160.0°C at 10.0 K/min
Heating schedule for sample 2-3:
   -40.0°C to 130.0°C at 10.0 K/min
   130.0°C to -40.0°C at -10.0 K/min
   -40.0°C to 130.0°C at 10.0 K/min
   130.0°C at -40.0°C at -10.0 K/min
   -40.0°C at 130.0°C at 10.0 K/min

The results are shown in Figure 11. The DSC data indicated that the T_{g} increases from ∼2,000 g/mol PBCA (70°C) to ∼20,000 g/mol PBCA (120°C). T_{g} for PBCA with a M_{w} of ∼200,000 g/mol (96°C) was found to be below the T_{g} for PBCA with a M_{w} ∼20,000 g/mol (120°C).

### EXAMPLE 5 Characterization of PBCA via Fourier Transform Infrared Spectroscopy (FTIR)

PBCA samples prepared as described in EXAMPLE 2 (samples 2-1, 2-2 and 2-3) were analyzed *via* FTIR using a Nicolet iS 10 device (Thermo Scientific) at an IR range of from 400-4000 cm⁻¹. Likewise, a PBCA having a Mw of about 2,000 g/mol was analyzed that was isolated from the milky suspension obtained from a BCA polymerization in water according to Curic et al. (Eur J Pharm Sci 78:121-131, 2015). The FTIR spectrum obtained for the PBCA sample synthesized by anionic polymerization in water exhibits a typical -OH stretch vibration at 3200-3500 cm⁻¹, whereas the FTIR spectra of the other samples (samples 2-1, 2-2, 2-3) did not. This finding indicates that the polymerization in water is initiated by a hydroxide group resulting in a hydroxyl terminated PBCA chain. In case of the other samples (samples 2-1, 2-2, 2-3), which were mainly manufactured in THF with only a small amount of water present, the polymerization is apparently initiated by a methoxide group and not by a hydroxide group.

### EXAMPLE 6 Preparation of PBCA-based nanoparticles

1 ml solvent phase (containing 20 mg PBCA and 5 mg Budesonide (16,17-(butylidene-bis(oxy))-11,21-dihydroxy-, (11-β,16-α)-pregna-1,4-diene-3,20-dione) in CHCl₃) was added to 2 ml of an aqueous phase (containing 10 mM sodium cholate and 0.01 mM polyoxyethylene (20) sorbitan monooleate (polysorbate 80) in deionized water). The mixture was emulsified using an UP200S probe sonicator (Hielscher Ultrasonics) (5 min, 70% amplitude, 1 cycle). The emulsion was stirred under a laboratory hood at room temperature for 1.5 h so as to evaporate the CHCl₃. The resulting nanoparticles suspension was passed through a filter having a pore size of 0.2 µm made from regenerated cellulose (Minisart® RC15 Syringe Filter, Sartorius). The size, and size distribution of the resulting nanoparticles as well as their concentration (nanoparticles per ml suspension) were determined by DLS and NTA as described above. The absolute drug loading of the nanoparticles was determined from the concentration of Budesonide within the nanoparticles *via* Reversed Phase High-Performance Liquid Chromatography (RP-HPLC) using an Agilent 1100 Series HPLC device (Agilent Technologies, Germany) (mobile phase: 67.5% acetonitrile + 32.% H₂O containing 0.1% TFA, flow: 0.5 ml/min, 11 MPa, column: Phenomenex Gemini NX C18, 3 µm mean particle size, 110 Å pore size) and detection at 243 nm. Based on this Budesonide concentration value, the absolute drug loading (AL) of the nanoparticles and the encapsulation efficiency (EE) was determined. For determining the molecular mass of the PBCA within the nanoparticles, the nanoparticle suspension was lyophilized, the lyphilisate then dissolved in THF and the solution analyzed using APC device (Waters) as described in EXAMPLE 1.

### EXAMPLE 7 Preparation of "empty" PBCA-based nanoparticles

"Empty" PBCA-based nanoparticles, i.e. nanoparticles without drug cargo (such as Budesonide), were prepared as described in EXAMPLE 6, apart from using a solvent phase containing 5 mg/ml or 10 mg/ml PBCA but no Budesonide. PBCA having a Mw of 2,618 +/- 22 g/mol (sample # 7-1), 17,162 +/- 60 g/mol (sample # 7-2) or 173,422 +/-2,777 g/mol (sample # 7-3) was used. The experiments were performed in triplicates (batches 1-3). Size and size distribution of the resulting nanoparticles were determined as described in EXAMPLE 6. Statistical analysis was performed by two-way analysis of variance (ANOVA). The results are shown in Figures 12-17. No significant size variability (ns) between the individual batches could be detected. Based on the detected low batch-to-batch variation, it was demonstrated that the nanoparticle manufacturing method is a repeatable and robust process. The experiment revealed that the nanoparticle diameter (mean or z-average) increased significantly with increasing M_{w} of the polymer.

### EXAMPLE 8 Stability of PBCA in aqueous nanoparticle suspensions

"Empty" PBCA-based nanoparticles were prepared as described in EXAMPLE 7. After CHCl₃ evaporation and filtration the nanoparticles were present as aqueous suspensions (cf. EXAMPLE 6). The M_{W} of the PBCA within the nanoparticles was determined by APC as described in EXAMPLE 1. The nanoparticle suspensions were kept for 7 days at room temperature (25°C). Then the M_{W} of the PBCA within the nanoparticles was determined again. The M_{w} of PBCA having an initial M_{W} of 2,618 g/mol or 17,162 g/mol (samples # 8-1 and # 8-2) remained basically the same after preparation and storage of the nanoparticles, while the M_{w} of PBCA having an initial M_{W} of 173,422 g/mol (sample # 8-3) dropped significantly (see Table 4).

**Table 4: Preparation of PBCA-based nanoparticles: Compositions of solvent phases**

| PBCA # | initial M_{w} [g/mol]* | M_{w} directly after nanoparticle preparation [g/mol]* | M_{w} 7 days after nanoparticle preparation [g/mol]* |
|---|---|---|---|
| 8-1 | 2,618 | 2,896 | 2,811 |
| | +/- 22 | +/- 83 | +/- 21 |
| 8-2 | 17,162 | 14,209 | 14,008 |
| | +/- 60 | +/- 222 | +/- 460 |
| 8-3 | 173,422 | 10,548 | 11,260 |
| | +/- 2,777 | +/- 33 | +/- 71 |

| | | | |
|---|---|---|---|
| n=3 | | | |

### EXAMPLE 9 Preparation of PBCA-based nanoparticles

PBCA-based nanoparticles were prepared as described in EXAMPLE 6, apart from using a solvent phase containing Budesonide at concentrations of 2 mg/ml or 5 mg/ml and PBCA at concentrations of 5 mg/ml or 10 mg/ml. PBCA having a M_{W} of 1,213 +/-21 g/mol (sample # 9-LMW), 12,436 +/- 25 g/mol (sample # 9-MMW) or 288,613 +/-1,518 g/mol (sample # 9-HMW) was used. An overview of the compositions of the solvent phases used in the experiments is shown in Table 5.

**Table 5: Preparation of PBCA-based nanoparticles: Compositions of solvent phases**

| Sample # | PBCA sample # | PBCA [mg/ml]* | Budesonide [mg/ml]* |
|---|---|---|---|
| 9-1 | 9-LMW | 5 | 2 |
| 9-2 | 9-LMW | 5 | 5 |
| 9-3 | 9-LMW | 10 | 2 |
| 9-4 | 9-LMW | 10 | 5 |
| 9-5 | 9-MMW | 5 | 2 |
| 9-6 | 9-MMW | 5 | 5 |
| 9-7 | 9-MMW | 10 | 2 |
| 9-8 | 9-MMW | 10 | 5 |
| 9-9 | 9-HMW | 5 | 2 |
| 9-10 | 9-HMW | 5 | 5 |
| 9-11 | 9-HMW | 10 | 2 |
| 9-12 | 9-HMW | 10 | 5 |

| | | | |
|---|---|---|---|
| * in CHCl₃ | | | |

Size, PDI and absolute drug loading of the resulting nanoparticles as well as encapsulation efficiency and the absolute Budesonide concentration in the nanoparticles suspension were determined as described in EXAMPLE 6. The results are shown in Table 6.

**Table 6: Preparation of PBCA-based nanoparticles: Results**

| Sample # | absolute drug loading (AL) [%] | Budesonide concentration [mg/ml] | mean particle size *via* DLS [nm] | encapsulation efficiency (EE) [%] | PDI |
|---|---|---|---|---|---|
| 9-1 | 5.2 | 0.26 | 79 | 13.1 | 0.129 |
| 9-2 | 5.1 | 0.26 | 66 | 5.1 | 0.131 |
| 9-3 | 3.8 | 0.39 | 85 | 19.2 | 0.134 |
| 9-4 | 4.4 | 0.44 | 85 | 8.9 | 0.112 |
| 9-5 | 17.4 | 0.87 | 111 | 43.5 | 0.160 |
| 9-6 | 17.9 | 0.90 | 105 | 17.9 | 0.100 |
| 9-7 | 20.9 | 2.09 | 121 | 104.7 | 0.113 |
| 9-8 | 18.3 | 1.83 | 119 | 36.5 | 0.108 |
| 9-9 | 15.7 | 0.79 | 123 | 39.3 | 0.090 |
| 9-10 | 13.5 | 0.68 | 119 | 13.5 | 0.099 |
| 9-11 | 7.5 | 0.75 | 144 | 37.7 | 0.102 |
| 9-12 | 6.1 | 0.61 | 139 | 12.2 | 0.096 |

In addition, the "design of experiment" methodology (DoE) was applied to these results in order to investigate the effect of varying PBCA M_{w} on the nanoparticle drug loading. The DoE methodology is frequently used in the pharmaceutical development in order to develop and optimize drug products or manufacturing processes. DoE methodology reduces required resources (such as time or staff) to gain the required information. In addition, it detects the impact and interactions between the investigated factors. In simple terms, DoE delivers (to the experimenter) the widest possible range of information with a minimal number of experiments without sacrificing a certain quality of results. DoE is also frequently used for the formulation or manufacturing process optimization in the nanoparticle field.

Based on the results in Table 6, the experimental design was generated and calculated using the JMP® v8.0 software (SAS Institute Inc., Cary, NC, USA), wherein a desirability function was generated for each parameter (where higher absolute drug load, higher encapsulation efficiency and lower particle size means higher desirability) and the overall desirability was calculated as the geometric mean of the desirability for each parameter. The calculated DoE results affirmed that a higher M_{w} of the polymer leads to an increased particle size of the nanoparticles. The particle size was not influenced by the amount of the drug substance or the amount of polymer. In all conditions, the PBCA-based nanoparticles had a narrow size distribution (PDI < 0.17) and the distribution was obviously not affected by any of the investigated factors. The absolute drug loading of the PBCA-based nanoparticles was increased for the MMW PBCA compared to the LMW or HMW PBCA. The drug concentration in the formulation was more or less doubled (∼2 mg/ml) and the AL significantly increased (∼20%). A higher PBCA amount increased the drug concentration and EE as well.

### EXAMPLE 10 Preparation of PBCA-based nanoparticles

PBCA-based nanoparticles were prepared as described in EXAMPLE 6, apart from using a solvent phase containing Budesonide at concentrations of 2.5 mg/ml, 5 mg/ml or 10 mg/ml and PBCA at concentrations of 10 mg/ml, 20 mg/ml or 40 mg/ml. PBCA having a M_{W} of 2,613 +/- 70 g/mol (sample # 10-LMW) or 12,760 +/- 144 g/mol (sample # 10-MMW) was used. The experiments were performed in triplicates. An overview of the compositions of the solvent phases used in the experiments is shown in Table 7. Size, PDI and absolute drug loading of the resulting nanoparticles as well as encapsulation efficiency and the absolute Budesonide concentration in the nanoparticles suspension were determined as described in EXAMPLE 6.

**Table 7: Preparation of PBCA-based nanoparticles: Compositions of solvent phases**

| Sample # | PBCA sample # | PBCA [mg/ml]* | Budesonide [mg/ml]* |
|---|---|---|---|
| 10-1 | 10-LMW | 10 | 2.5 |
| 10-2 | 10-LMW | 20 | 5.0 |
| 10-3 | 10-LMW | 40 | 10.0 |
| 10-4 | 10-MMW | 10 | 2.5 |
| 10-5 | 10-MMW | 20 | 5.0 |
| 10-6 | 10-MMW | 40 | 10.0 |

| | | | |
|---|---|---|---|
| * in CHCl₃ | | | |

Statistical analysis was performed by two-way analysis of variance (ANOVA). The results are shown in Figures 18-20 and Table 8.

**Table 8: Preparation of PBCA-based nanoparticles: Results**

| Sample # | absolute drug loading (AL) [%] | Budesonide concentration [mg/ml] | mean particle size *via* DLS [nm] | encapsulation efficiency (EE) [%] | PDI |
|---|---|---|---|---|---|
| 10-1 | 6.46 +/- 1.11 | 0.65 +/- 0.11 | 100 +/- 3 | 26 +/- 5 | 0.119 +/- 0.006 |
| 10-2 | 3.54 +/- 0.12 | 0.71 +/- 0.02 | 103 +/-2 | 14 +/- 1 | 0.123 +/- 0.003 |
| 10-3 | 3.48 +/- 0.44 | 1.39 +/- 0.17 | 106 +/- 0 | 14 +/- 2 | 0.126 +/- 0.013 |
| 10-4 | 18.16 +/- 2.18 | 1.82 +/- 0.22 | 109 +/- 6 | 73 +/- 12 | 0.108 +/- 0.011 |
| 10-5 | 8.79 +/- 1.03 | 1.76 +/- 0.21 | 117 +/- 3 | 35 +/- 5 | 0.114 +/- 0.004 |
| 10-6 | 7.97 +/- 1.08 | 3.19 +/- 0.43 | 128 +/- 0 | 32 +/- 5 | 0.103 +/- 0.005 |

MMW PBCA (# 10-4, #10-5 and #10-6) significantly increased the drug concentration and AL compared to LMW PBCA (#10-1, #10-2, #10-3). The absolute drug concentration in the liquid formulation was increased up to -3.5 mg/ml using 40 mg/ml of the MMW PBCA.

### EXAMPLE 11 Toxicity of PBCA-based nanoparticles: Pre-experiment for assessing the toxicity of sodium cholate and polysorbate 80

CACO-2 cells (DSMZ ACC-169) were grown in cell culture flasks at 37°C and 5% CO₂. The culture medium was Dulbecco's modified Eagle's medium (DMEM) containing 25 mM glucose, 4 mM glutamine, 100 U/ml penicillin, 100 U/ml streptomycin and 10 vol-% fetal bovine serum (FBS). The cells were washed with sterile Dulbecco's phosphate buffered saline (DPBS, free of Ca²⁺ and Mg²⁺) and detached from the flask using phosphate buffered saline (PBS) containing trypsin and 10 mM ethylenediaminetetraacetic acid (EDTA). Cells were sown into 96 well cell culture plates at a density of about 10,000 cells/well and kept for about 24 h in culture medium at 37°C and 5% CO₂. All conditions (samples and controls) were tested in 8 wells each as follows:
Samples and negative control (100% culture medium, 0% toxicity):
   50 µl/well sample/negative control, 6 h at 37°C and 5% CO₂
Positive control (100% DMSO, 100% toxicity):
   50 µl/well positive control, 5 min at 37°C and 5% CO₂

A summary of the sample conditions is shown in Table 9.

**Table 9: Conditions tested in toxicity assay**

| Sample # | sodium cholate [mM]* | polysorbate 80 [µM]* |
|---|---|---|
| 11-1 | 0.039 | 0.039 |
| 11-2 | 0.078 | 0.078 |
| 11-3 | 0.156 | 0.156 |
| 11-4 | 0.313 | 0.313 |
| 11-5 | 0.625 | 0.625 |
| 11-6 | 1.250 | 1.250 |
| 11-7 | 2.500 | 2.500 |
| 11-8 | 5.000 | 5.000 |
| 11-9 | 7.500 | 7.500 |
| 11-10 | 10.000 | 10.000 |

| | | |
|---|---|---|
| *in culture medium | | |

Cell viability was assessed using MTT (3-[4,5-dimethylthiazol-2-yl]-2,5-diphenyltetrazolium bromide; thiazolyl blue) by adding 15 µl/well of 5 mg/ml MTT in DPBS, incubating for 6 h at 37°C and 5% CO₂, aspirating the liquids from the cells, adding 100 µl/well DMSO, incubating for 15 min in the dark while shaking and measuring the absorbance at 570 nm (reference wavelength: 690 nm).

The results are shown in Figure 21. With samples #11-1 to #11-7 (i.e. up to a concentration of 2.5 mM sodium cholate and 2.5 µM polysorbate 80) the cells did not show any significant reduction in viability.

### EXAMPLE 12 Toxicity of PBCA-based nanoparticles

"Empty" PBCA-based nanoparticles were prepared as described in EXAMPLE 7 (with 10 mg/ml PBCA in the solvent phase). The nanoparticle preparations contained final concentrations of about 2.5 mM sodium cholate and about 2.5 µM polysorbate (after a 1:4 dilution). All conditions (samples and controls) were tested in 8 wells each as described for EXAMPLE 11. Also, the same controls were used: positive control (100% DMSO, 100% toxicity); negative control (100% culture medium, 0% toxicity). A sample without nanoparticles corresponding to sample #11-7 (cf. Table 9) was included as a further negative control. A summary of the nanoparticle samples is shown in Table 10, wherein the amount of nanoparticles is expressed as the amount of PBCA contained in the sample. The experiment was performed 3 times, each time with freshly prepared nanoparticle preparations. Statistical analysis was performed by two-way analysis of variance (ANOVA).

**Table 10: Conditions tested in toxicity assay**

| Sample # | PBCA [µg/ml]* |
|---|---|
| negative control (0% toxicity) | 0 |
| 12-1 | 10 |
| 12-2 | 20 |
| 12-3 | 39 |
| 12-4 | 78 |
| 12-5 | 156 |
| 12-6 | 313 |
| 12-7 | 625 |
| 12-8 | 1250 |
| 12-9 | 2500 |

| | |
|---|---|
| *in culture medium | |

The results are shown in Figure 22. In particular at high concentrations, nanoparticles prepared from mid-Mw PBCA (∼20,000 g/mol) were significantly less toxic than nanoparticles prepared from low-Mw PBCA (∼2,000 g/mol).

### EXAMPLE 13 PBCA end capping

The C-H acidic end group in the PBCA chain was capped with ethyl 2-(bromomethyl) acrylate (EBMA) resulting in a terminated PBCA (tPBCA) molecule as described by Kohsaka et al. (Macromol Chem Phys 216(14):1534-1539, 2015). The polymerization procedure was similar to the method described in EXAMPLE 2 except that the terminator molecule EBMA was added to the polymerization mixture.

5 ml of 2 mM TFA in THF was mixed with BCA while stirring to a final concentration of 326 mM BCA. Polymerization was started by adding 120 µl of an aqueous SMeO solution so as to obtain a final concentration of 8 mM or 80 mM SMeO. Stirring was continued for 3 h. 2.5 ml of 50 mM or 10 mM EBMA solution (diluted in THF) was mixed with 2.5 ml of PBCA containing polymerization mixture resulting in a final EBMA concentration of 25 mM or 5 mM and a final BCA concentration of 163 mM. The EBMA and PBCA containing solution were additionally stirred for 1 h. Then, the PBCA containing reaction mixture was precipitated, purified as described in EXAMPLE 2 and subsequently lyophilized as described in EXAMPLE 1. For the M_{w} characterization, PBCA was dissolved in THF to a final concentration of 5 mg/ml and the mass-average molecular mass (M_{W}) and the number-average molecular mass (Mₙ) of the polymer (and thus also the dispersity Ð_{M} = M_{W} / Mₙ thereof) were determined by GPC.

### EXAMPLE 14 Nuclear magnetic resonance spectroscopy

Nuclear magnetic resonance spectroscopy (NMR) was used to verify the chemical structure of PBCA or tPBCA after the manufacturing process. Additionally, NMR spectra of BCA monomer were also recorded and compared to the polymer spectra in order to detect a potential monomer residue. 600 MHz ¹H and 150 MHz ¹³C NMR spectra were obtained with a Bruker Avance 600 MHz system (Karlsruhe, Deutschland).

### ¹³C and ¹H NMR spectra of PBCA

PBCA having two different M_{w} (∼2,000 g/mol and ∼20,000 g/mol) was analyzed by ¹³C and ¹H measurements. Additionally, ¹³C and ¹H NMR spectra of BCA monomer were recorded in order to explore potential monomer residues in the polymer. The ¹³C and ¹H NMR-signals of the terminal olefinic =CH₂ group of the BCA monomer disappeared almost completely, i.e. could only be detected in trace amounts, indicating the successful polymerization to PBCA and the essential absence of the BCA monomer.

### ¹³C and ¹H NMR spectra of tPBCA

In addition, the structure of ethyl 2-(bromomethyl) acrylate (EBMA) capped PBCA was analyzed by NMR (tPBCA). The ethyl 2-(bromomethyl) acrylate capped PBCA (tPBCA) was prepared according to EXAMPLE 13. PBCA having a M_{w} of ∼2,000 g/mol and ∼20,000 g/mol was terminated with two EBMA concentrations, 25 mM or 5 mM. ¹³C and ¹H NMR spectra of tPBCA were recorded in order to verify the successful linking between the polymer and the termination molecule EBMA. The ¹³C and ¹H NMR-signals of the methylene group (-CH₂-) in α-position to the bromine and the ¹³C and ¹H NMR-signals for the terminal olefinic =CH₂ group of EBMA were only present in minor amounts or disappeared completely, indicating that most or all of the added EBMA had reacted. Additionally, a comparison of the ¹³C and ¹H NMR spectra of PBCA and tPBCA confirmed that the PBCA backbone structure was not changed after the addition of EBMA. In the ¹³C and ¹H NMR spectra of tPBCA having M_{w} of ∼2,000 g/mol and ∼20,000 g/mol, terminated with 25 mM EBMA, and in the ¹³C and ¹H NMR spectra of tPBCA having M_{w} of ∼20,000 g/mol, terminated with 5 mM EBMA, the original signal of the terminal olefinic =CH₂ group of EBMA was still detectable revealing the presence of unreacted EBMA molecules. This means that in these cases an excess of the EBMA terminator was added to PBCA. In the ¹³C and ¹H measurements of 5 mM EBMA the original signal of the terminal olefinic =CH₂ group of EBMA was not found in the ∼2,000 g/mol tPBCA indicating a complete reaction between EBMA and polymer.

These results confirmed that the terminator amount required for a desired degree of termination, e.g. for complete reaction of all polymer chains with the terminator compound, depends on the polymer M_{w} and thus on the number of polymer chain termini. In an equal polymer mass, a higher M_{w} has a smaller number of chain termini compared to a lower M_{w}. Consequently, the lower the PBCA M_{w} the higher the required terminator amount to block all chain termini.

The ¹H NMR signal of the terminal olefinic =CH₂ group of EBMA was also used to demonstrate the successful reaction between the EBMA molecule and the polymer chain terminus. The chemical shifts of the terminal olefinic =CH₂ protons of EBMA changed upon reaction with the C-H acidic terminus of PBCA, which confirmed that the vinyl protons are integrated in another molecule. Consequently, it confirmed the linkage between PBCA and the EBMA molecule.

In addition, the ¹H spectra for the tPBCA samples showed also trace amounts of monomer residues, as described above for the not terminated PBCA. The monomer residues probably originated from the SMeO forced monomer unzipping reaction during the depolymerization/repolymerization before the terminator EBMA was added.

### EXAMPLE 15 Monomer release in solution

Based on the detected presence of the monomer in PBCA and tPBCA, a chemical stability study was performed in order to monitor possible monomer release in PBCA and tPBCA during storage in solution. The relative monomer content after the production and the relative monomer release of the polymer during storage in solution (in CDCl₃) were monitored for PBCA and tPBCA. PBCA was terminated with two different ethyl 2-(bromomethyl) acrylate (EBMA) concentrations, 5 mM and 25 mM. Approximately 5 mg tPBCA and PBCA having a M_{w} of ∼2,000 g/mol and ∼20,000 g/mol was dissolved in 600 µl CDCl₃ and stored for 7 weeks at room temperature protected from light in a closed glass vial. 600 MHz ¹H NMR spectrum was recorded once a week using a Bruker Avance NMR spectrometer (Karlsruhe, Deutschland). The monomer content was estimated using the butylated hydroxytoluene (BHT) signal (6.98 ppm) as reference. BHT has a stabilizing function in the polymerization medium THF (prevents peroxide formation during THF storage). Thus, the signal coming from the synthesis residual BHT in the polymer was assumed to be constant (integral BHT = constant =1). Consequently, the integral of the monomer signal (7.058 ppm) created by the vinyl group was calculated according to the equation: ${Integral}_{monomer} = \left(\frac{Peak area {monomer}_{7.058 ppm}}{Peak area {BHT}_{6.98 ppm}}∗{Integral}_{BHT}\right)$

The obtained value for Intergralₘₒₙₒₘₑᵣ describes the relative area of the signal at 7.058 ppm. Hence, an increasing Integralₘₒₙₒₘₑᵣ value indicates an increasing peak area and therewith an increasing monomer amount.

Experimental results are displayed in Figures 23 and 24. First, the results demonstrated that the monomer release is indeed a progressive phenomenon for PBCA. Second, the results confirmed that the EBMA capping of the PBCA chains (tPBCA) inhibited the monomer release. The monomer amount for not terminated PBCA chains (∼2,000 g/mol and ∼20,000 g/mol) increased clearly during the storage time. This indicated that the PBCA chain is truly "living" and as consequence was able to release monomer units by an unzipping reaction from the chain termini. In comparison, the monomer amount of tPBCA was constant during the storage time of 7 weeks for both terminator concentrations (5 mM and 25 mM) in both M_{w} ranges (∼2,000 g/mol and ∼20,000 g/mol). In fact, the termination with EBMA deprived the PBCA chain of their living character and prevented the monomer release.

### EXAMPLE 16 Toxicity of tPBCA-based nanoparticles

The toxicity of tPBCA-based nanoparticles was determined as described in EXAMPLE 12 using PBCA and tPBCA as polymer matrix in the nanoparticles at two M_{w} ranges, ∼2,000 g/mol and ∼20,000 g/mol. The results are depicted in Figures 25 and 26. As can be seen from these Figures, the cytotoxic effect was reduced for tPBCA-based nanoparticles. In both M_{w} ranges (∼2,000 g/mol and ∼20,000 g/mol) the cytotoxicity was significantly decreased for the tPBCA-based nanoparticles at different polymer concentrations. In general, the study evidenced that the termination of the PBCA chain termini increased the viability and consequently decreased the cytotoxicity of the nano-formulation.

### EXAMPLE 17 Impedance spectroscopy of PBCA-based nanoparticles

The barrier integrity of CACO-2 cells after treatment with "empty" PBCA- and tPBCA-based nanoparticles consisting of two different M_{w} was analyzed in order to determine the impact of the polymer matrix on the cell barrier tightness. The destruction of cell barriers by monitoring the barrier tightness is frequently used to analyze the potential cytotoxicity of nanoparticle formulations (Kolter et al. J Control Release 197:165-79, 2015).

The barrier integrity was monitored by measuring the transendothelial electrical resistance (TEER) using the CellZscope device from nanoAnalytics (Münster, Germany). PBCA and tPBCA were manufactured with 8 mM and 80 mM SMeO as described in EXAMPLE 2 or in EXAMPLE 13 and subsequently "empty" nanoparticles consisting of two different M_{w} (∼2,000 g/mol and ∼20,000 g/mol) were produced as described in EXAMPLE 6 with a final PBCA amount of 10 mg/ml.

The impedance spectroscopy assays were carried out as follows. Cells were seeded in a costar® Transwell® insert at a density of 3*10⁵ cell/cm². The cells were cultivated for 14 days and the medium was changed in the apical and basolateral compartment three times a week in the first 7 days and afterwards daily. After 14 days of cultivation, 24 Transwell® filter inserts were transferred in the CellZscope device and the cells were additionally cultivated for 48 h inside of the device. The absolute TEER value was measured for 48 h in order to monitor the cell equilibration and the formation of monolayers with a stable TEER value within the device. Only cell monolayers with a TEER value of 150-250 Ω*cm² were used for the assay. Freshly prepared empty PBCA-based nanoparticles were then diluted in DMEM to defined PBCA concentrations (Table 11).

**Table 11: PBCA concentrations tested in impedance spectroscopy assay**

| PBCA concentration [µg/ml] |
|---|
| 2,000 |
| 1,000 |
| 500 |
| 250 |
| 100 |
| 25 |
| 2.5 |
| 0.25 |

After removing the old medium in the apical compartment and replacing DMEM in the basolateral compartment, 250 µl empty PBCA-based nanoparticles PBCA and tPBCA were spiked to the apical compartment. 250µl DMEM was used as negative control. The experiment was performed in duplicates and the TEER value was measured for 24 h. Based on the measured TEER values the relative TEER value (TEERᵣ) was calculated according to the following equation: ${TEER}_{r} \left(%\right) = \frac{{TEER}_{t}}{{TEER}_{initial}} ∗ 100$

TEERᵢₙᵢₜᵢₐₗ is the starting TEER value in Ω*cm² after 48 h culturing and equilibrating in the CellZscope device. TEERₜ is the TEER value measured every hour at the time point t.

The results for the PBCA concentrations of 250 µg/ml and 1000 µg/ml are shown in Figures 27 and 28. The data reveal a PBCA M_{w}- and concentration-dependent effect on the barrier integrity of CACO-2 cells. In particular at high PBCA concentrations (of 1000 µg/ml and above), ∼2,000 g/mol PBCA-based nanoparticles induced a considerably stronger TEER breakdown compared to ∼20,000 g/mol PBCA-based nanoparticles. In the event of 250 µg/ml PBCA-based nanoparticles, ∼20,000 g/mol PBCA-based nanoparticles did not affect the barrier integrity (100%), whereas ∼2,000 g/mol PBCA-based nanoparticles reduced the TEER value to 50%. No distinct barrier opening was observed for nanoparticle concentrations below 250 µg/ml (100 µg/ml-0.25 µg/ml).

## Claims

1. A nanoparticle comprising:
(1) one or more than one polymer comprising from 60 to 330 monomeric constituents selected from C₁-C₁₀-alkyl cyanoacrylates and C₁-C₆-alkoxy-C₁-C₁₀-alkyl cyanoacrylates, and having a dispersity of less than 3.0, and
(2) one or more than one cargo compound selected from pharmaceutically active agents, cosmetically active agents and nutritional supplements.

2. The nanoparticle of claim 1, wherein the polymer(s) form(s) a polymeric matrix, and the cargo compound(s) is/are incorporated in the polymeric matrix, or wherein the polymer(s) form(s) a polymeric shell, and the cargo compound(s) is/are comprised in a core encapsulated by said polymeric shell.

3. The nanoparticle of claim 1 or claim 2, wherein the solubility of the cargo compound(s) in water at 25°C and at pH 7.0 is 1 g/l or less.

4. The nanoparticle of any one of claims 1-3, wherein the polymer(s) is/are selected from poly(n-butyl 2-cyanoacrylates), poly(isobutyl 2-cyanoacrylates), poly(*sec-*butyl 2-cyanoacrylates), poly(*tert*-butyl 2-cyanoacrylates), poly(ethyl 2-cyanoacrylates) and poly(n-octyl 2-cyanoacrylates).

5. The nanoparticle of any one of claims 1-4, wherein the nanoparticle further comprises one or more than one nanoparticle-stabilizing agent selected from the bile acids cholic acid, taurocholic acid, glycocholic acid, deoxycholic acid, lithocholic acid, chenodeoxycholic acid, dehydrocholic acid, ursodeoxycholic acid, hyodeoxycholic acid and hyocholic acid, salts of said bile acids, and mixtures of said bile acids and/or said bile salts, the amount of the nanoparticle-stabilizing agent(s) preferably being from 3 to 36 wt-% relative to the total mass of the polymer(s) and cargo compound(s) of the nanoparticle.

6. The nanoparticle of any one of claims 1-5, wherein the diameter of the nanoparticle is less than 500 nm.

7. The nanoparticle of any one of claims 1-6, further comprising one or more than one uptake mediator selected from polyoxyethylene sorbitan fatty acid esters.

8. The nanoparticle of any one of claims 1-7, further comprising a sorbitan fatty acid ester.

9. The nanoparticle of any one of claims 1-8, where the C-H acidic end(s) of the polymer(s) is/are functionalized with a linear or branched C₁-C₁₀-alkyl radical or a linear or branched C₂-C₁₀-alkenyl radical comprising at least one double bond of which at least one is terminal, where the C₁-C₁₀-alkyl radical and the C₂-C₁₀-alkenyl radical independently of each other are unsubstituted or substituted by one or more substituent(s) selected from C₁-C₁₀-alkoxy, C₁-C₁₀-alkylcarbonyl, C₁-C₁₀-alkoxycarbonyl and nitro, preferably a linear or branched C₂-C₆-alkenyl radical comprising a terminal double bond, where the C₂-C₆-alkenyl radical is unsubstituted or substituted with C₁-C₁₀-alkoxycarbonyl, and more preferably 2-(C₁-C₁₀-alkoxycarbonyl)allyl.

10. A pharmaceutical composition comprising a plurality of nanoparticles of any one of claims 1-9, and a pharmaceutically acceptable carrier.

11. A method for preparing a polymer having a defined degree of polymerization, the method comprising:
(i) providing a liquid phase comprising monomer selected from C₁-C₁₀-alkyl cyanoacrylates and C₁-C₆-alkoxy-C₁-C₁₀-alkyl cyanoacrylates dissolved in an aprotic water-miscible organic solvent or a mixture of two or more aprotic water-miscible organic solvents, where the liquid phase may moreover comprise one or more than one C₁-C₃-alkanol;
(ii) adding one or more than one base selected from metal hydroxides and metal alkoxides to the liquid phase, where, in case that the base is selected from metal hydroxides and the liquid phase provided in (i) does not contain any C₁-C₃-alkanols, the metal hydroxide(s) is/are added in the presence of one or more than one C₁-C₃-alkanol;
(iii) allowing an equilibrium of depolymerization and repolymerization processes to establish such that the polymer having a defined degree of polymerization is formed; and
(iv) quenching the polymerization reaction.

12. The method of claim 11, wherein the quenching in step (iv) involves mixing the liquid phase obtained in step (iii) containing the polymer having the defined degree of polymerization with an aqueous acid solution so as to precipitate the polymer.

13. The method of claim 11 or claim 12, wherein the liquid phase obtained in step (iii) containing the polymer having a defined degree of polymerization is reacted with a terminator compound of the general formula X-R³, wherein X is a leaving group and R³ is a linear or branched C₁-C₁₀-alkyl radical or a linear or branched C₂-C₁₀-alkenyl radical comprising at least one double bond of which at least one is terminal, where the C₁-C₁₀-alkyl radical and the C₂-C₁₀-alkenyl radical independently of each other are unsubstituted or substituted by one or more substituent(s) selected from C₁-C₁₀-alkoxy, C₁-C₁₀-alkylcarbonyl, C₁-C₁₀-alkoxycarbonyl and nitro, prior to the quenching in step (iv), wherein preferably X is a halogen and R³ is a linear or branched C₂-C₆-alkenyl radical comprising a terminal double bond, where the C₂-C₆-alkenyl radical is unsubstituted or substituted with C₁-C₁₀-alkoxycarbonyl; more preferably the terminator compound is C₁-C₁₀-alkyl 2-bromomethyl acrylate.

14. A method for preparing nanoparticles, the method comprising:
(a) providing a hydrophobic liquid phase comprising:
(1) one or more than one polymer comprising from 60 to 330 monomeric constituents selected from C₁-C₁₀-alkyl cyanoacrylates and C₁-C₆-alkoxy-C₁-C₁₀-alkyl cyanoacrylates, and having a dispersity of less than 3.0, and
(2) one or more than one cargo compound selected from pharmaceutically active agents, cosmetically active agents and nutritional supplements
dissolved in a water-immiscible organic solvent or a mixture of two or more water-immiscible organic solvents;
(b) providing a hydrophilic liquid phase, the hydrophilic liquid phase preferably comprising one or more than one of nanoparticle-stabilizing agent selected from bile acids, salts of bile acids, and mixtures thereof, and the nanoparticle-stabilizing agent(s) being dissolved in a hydrophilic solvent;
(c) finely dispersing the hydrophobic liquid phase in the hydrophilic liquid phase so as to form an emulsion; and
(d) removing at least part of the organic solvent(s) from the emulsion so as to obtain a suspension of nanoparticles in the hydrophilic solvent.

15. A nanoparticle obtainable by the method of claim 14.

## Patentansprüche

1. Nanopartikel, umfassend:
(1) ein oder mehr als ein Polymer mit 60 bis 330 Monomer-Einheiten, ausgewählt unter C₁-C₁₀-Alkylcyanoacrylaten und C₁-C₆-Alkoxy-C₁-C₁₀-alkylcyanoacrylaten, und einer Dispersität von weniger als 3,0, und
(2) eine oder mehr als eine Cargo-Verbindung, ausgewählt unter pharmazeutisch aktiven Wirkstoffen, kosmetisch aktiven Wirkstoffen und Nahrungsergänzungsmitteln.

2. Nanopartikel nach Anspruch 1, wobei das Polymer (die Polymere) eine polymere Matrix bildet/bilden, und die Cargo-Verbindung(en) in die polymere Matrix integriert ist/sind, oder wobei das Polymer (die Polymere) eine polymere Hülle bildet/bilden, und die Cargo-Verbindung(en) in einem von dieser polymeren Hülle eingekapselten Kern enthalten ist/sind.

3. Nanopartikel nach Anspruch 1 oder Anspruch 2, wobei die Löslichkeit der Cargo-Verbindung(en) in Wasser 1 g/l oder weniger bei 25°C und pH 7.0 beträgt.

4. Nanopartikel nach einem der Ansprüche 1-3, wobei das Polymer (die Polymere) ausgewählt ist/sind unter Poly(n-butyl-2-cyanoacrylaten), Poly(isobutyl-2-cyanoacrylaten), Poly(*sec*-butyl-2-cyanoacrylaten), Poly(*tert*-butyl-2-cyanoacrylaten), Poly(ethyl-2-cyanoacrylaten) und Poly(n-octyl-2-cyanoacrylaten).

5. Nanopartikel nach einem der Ansprüche 1-4, wobei das Nanopartikel weiterhin ein oder mehr als ein Nanopartikel-stabilisierendes Mittel umfasst, ausgewählt unter den Gallensäuren Cholsäure, Taurocholsäure, Glycocholsäure, Desoxycholsäure, Lithocholsäure, Chenodeoxycholsäure, Dehydrocholsäure, Ursodeoxycholsäure, Hyodeoxycholsäure und Hyocholsäure, Salzen dieser Gallensäuren, und Mischungen dieser Gallensäuren und/oder dieser Gallensalze, wobei die Menge an Nanopartikel-stabilisierendem Mittel (Nanopartikelstabilisierenden Mitteln) vorzugsweise 3 bis 36 Gew.-% beträgt, bezogen auf die Gesamtmasse des Polymers (der Polymere) und der Cargo-Verbindung(en) des Nanopartikels.

6. Nanopartikel nach einem der Ansprüche 1-5, wobei der Durchmesser des Nanopartikels weniger als 500 nm beträgt.

7. Nanopartikel nach einem der Ansprüche 1-6, ferner umfassend einen oder mehr als einen Aufnahme-Mediator ausgewählt unter Polyoxyethylensorbitan-Fettsäureestern.

8. Nanopartikel nach einem der Ansprüche 1-7, ferner umfassend einen SorbitanFettsäureester.

9. Nanopartikel nach einem der Ansprüche 1-8, wobei das azide C-H-Ende des Polymers (die aziden C-H-Enden der Polymere) funktionalisiert ist/sind mit einem linearen oder verzweigten C₁-C₁₀-Alkylrest oder einem linearen oder verzweigten C₂-C₁₀-Alkenylrest mit mindestens einer Doppelbindung, von denen mindestens eine endständig ist, wobei der C₁-C₁₀-Alkylrest und der C₂-C₁₀-Alkenylrest unabhängig voneinander unsubstituiert oder durch einen oder mehrere Substituenten substituiert sind, ausgewählt unter C₁-C₁₀-Alkoxy, C₁-C₁₀-Alkylcarbonyl, C₁-C₁₀-Alkoxycarbonyl und Nitro, vorzugsweise einem linearen oder verzweigten C₂-C₆-Alkenylrest mit einer endständigen Doppelbindung, wobei der C₂-C₆-Alkenylrest unsubstituiert oder mit C₁-C₁₀-Alkoxycarbonyl substituiert ist, und stärker bevorzugt mit 2-(C₁-C₁₀-Alkoxycarbonyl)allyl.

10. Pharmazeutische Zusammensetzung, umfassend mehrere Nanopartikel nach einem der Ansprüche 1-9, und einen pharmazeutisch geeigneten Träger.

11. Verfahren zur Herstellung eines Polymers mit einem definierten Polymerisationsgrad, bei dem man:
(i) eine Flüssigphase bereitstellt, welche ein unter C₁-C₁₀-Alkylcyanoacrylaten und C₁-C₆-Alkoxy-C₁-C₁₀-alkylcyanoacrylaten ausgewähltes Monomer umfasst, das gelöst in einem aprotischen, mit Wasser mischbaren organischen Lösungsmittel oder einer Mischung von zwei oder mehr aprotischen, mit Wasser mischbaren organischen Lösungsmitteln ist, wobei die Flüssigphase außerdem ein oder mehr als ein C₁-C₃-Alkanol enthalten kann;
(ii) eine oder mehr als eine Base, ausgewählt unter Metallhydroxiden und Metallalkoxiden, zur Flüssigphase zugibt, wobei für den Fall, dass die Base unter Metallhydroxiden ausgewählt ist und die in (i) bereitgestellte Flüssigphase keinerlei C₁-C₃-Alkanole enthält, das Metallhydroxid (die Metallhydroxide) in Gegenwart von einem oder mehr als einem C₁-C₃-Alkanol zugegeben wird/werden;
(iii) ein Gleichgewicht von Depolymerisations- und Repolymerisations-Prozessen einstellen lässt, so dass das Polymer mit einem definierten Polymerisationsgrad gebildet wird; und
(iv) die Polymerisationsreaktion quencht.

12. Verfahren nach Anspruch 11, wobei man zum Quenchen in Schritt (iv) die in Schritt (iii) erhaltene Flüssigphase, die das Polymer mit dem definierten Polymerisationsgrad enthält, mit einer wässrigen Säurelösung mischt, um das Polymer auszufällen.

13. Verfahren nach Anspruch 11 oder Anspruch 12, wobei die in Schritt (iii) erhaltene Flüssigphase, die das Polymer mit dem definierten Polymerisationsgrad enthält, vor dem Quenchen in Schritt (iv) mit einer terminierenden Verbindung der allgemeinen Formel X-R³ umgesetzt wird, wobei X für eine Abgangsgruppe steht und R³ für einen linearen oder verzweigten C₁-C₁₀-Alkylrest oder einen linearen oder verzweigten C₂-C₁₀-Alkenylrest mit mindestens einer Doppelbindung, von denen mindestens eine endständig ist, steht, wobei der C₁-C₁₀-Alkylrest und der C₂-C₁₀-Alkenylrest unabhängig voneinander unsubstituiert oder durch einen oder mehrere Substituenten substituiert sind, ausgewählt unter C₁-C₁₀-Alkoxy, C₁-C₁₀-Alkylcarbonyl, C₁-C₁₀-Alkoxycarbonyl und Nitro, wobei vorzugsweise X für ein Halogen und R³ für einen linearen oder verzweigten C₂-C₆-Alkenylrest mit einer terminalen Doppelbindung steht, worin der C₂-C₆-Alkenylrest unsubstituiert oder mit C₁-C₁₀-Alkoxycarbonyl substituiert ist; und stärker bevorzugt die terminierende Verbindung für C₁-C₁₀-Alkyl-2-brommethylacrylat steht.

14. Verfahren zur Herstellung von Nanopartikeln, bei dem man:
(a) eine hydrophobe Flüssigphase bereitstellt, welche umfasst:
(1) ein oder mehr als ein Polymer mit 60 bis 330 Monomer-Einheiten, ausgewählt unter C₁-C₁₀-Alkylcyanoacrylaten und C₁-C₆-Alkoxy-C₁-C₁₀-alkylcyanoacrylaten, und einer Dispersität von weniger als 3,0, und
(2) eine oder mehr als eine Cargo-Verbindung, ausgewählt unter pharmazeutisch aktiven Wirkstoffen, kosmetisch aktiven Wirkstoffen und Nahrungsergänzungsmitteln,
gelöst in einem mit Wasser nicht mischbaren organischen Lösungsmittel oder einer Mischung aus zwei oder mehreren mit Wasser nicht mischbaren organischen Lösungsmitteln;
(b) eine hydrophile Flüssigphase bereitstellt, die vorzugsweise ein oder mehr als ein unter Gallensäuren, Salzen von Gallensäuren, und Mischungen davon ausgewähltes Nanopartikel-stabilisierendes Mittel umfasst, das in einem hydrophilen Lösungsmittel gelöst ist;
(c) die hydrophobe Flüssigphase fein in der hydrophilen Flüssigphase dispergiert, um eine Emulsion zu bilden; und
(d) zumindest einen Teil des organischen Lösungsmittels (der organischen Lösungsmittel) aus der Emulsion entfernt, um eine Suspension von Nanopartikeln im hydrophilen Lösungsmittel zu erhalten.

15. Nanopartikel, erhältlich nach dem Verfahren nach Anspruch 14.

## Revendications

1. Nanoparticule, comprenant :
(1) un ou plus d'un polymère comprenant de 60 à 330 constituants monomériques sélectionnés parmi : C₁-C₁₀-alkyle cyanoacrylates et C₁-C₆-alkoxy-C₁-C₁₀-alkyle cyanoacrylates, et ayant une dispersité inférieure à 3,0, et
(2) un ou plus d'un composé transporteur sélectionné parmi : des agents pharmaceutiquement actifs, des agents cosmétiquement actifs et des suppléments nutritifs.

2. Nanoparticule selon la revendication 1, dans laquelle le(s) polymère(s) forme(nt) une matrice polymérique, et les composé(s) transporteur(s) est/sont incorporé(s) dans la matrice polymérique, ou dans laquelle le(s) polymère(s) forme(nt) une enveloppe polymérique, et les composé(s) transporteur(s) est/sont compris dans un noyau encapsulé par ladite enveloppe polymérique.

3. Nanoparticule selon la revendication 1 ou la revendication 2, dans laquelle la solubilité des composé(s) transporteur(s) dans l'eau à 25°C et à un pH de 7,0 est d'1 g/l ou moins.

4. Nanoparticule selon l'une quelconque des revendications 1-3, dans laquelle le(s) polymère(s) est/sont sélectionné(s) parmi : poly(n-butyl 2-cyanoacrylates), poly(isobutyl 2-cyanoacrylates), poly(*sec*-butyl 2-cyanoacrylates), poly(*tert*-butyl 2-cyanoacrylates), poly(éthyl 2-cyanoacrylates) et poly(n-octyl 2-cyanoacrylates).

5. Nanoparticule selon l'une quelconque des revendications 1 à 4, dans laquelle la nanoparticule comprend en outre un ou plus d'un agent stabilisateur de nanoparticule sélectionné parmi : les acides biliaires acide cholique, acide taurocholique, acide glycocholique, acide désoxycholique, acide lithocholique, acide chénodésoxycholique, acide déhydrocholique, acide ursodésoxycholique, acide hyodéoxycholique et acide hyocholique, des sels desdits acides biliaires, et des mélanges desdits acides biliaires et/ou desdits sels biliaires, la quantité de l'agent (des agents) stabilisateur(s) de nanoparticule étant de préférence de 3 à 36 % en poids relativement à la masse totale du(des) polymère(s) et du(des) composé(s) transporteur(s) de la nanoparticule.

6. Nanoparticule selon l'une quelconque des revendications 1 à 5, dans laquelle le diamètre de la nanoparticule est inférieur à 500 nm.

7. Nanoparticule selon l'une quelconque des revendications 1 à 6, comprenant en outre un ou plus d'un médiateur d'absorption sélectionné parmi des esters d'acide gras de polyoxyéthylène sorbitane.

8. Nanoparticule selon l'une quelconque des revendications 1 à 7, comprenant en outre un ester acide gras de sorbitane.

9. Nanoparticule selon l'une quelconque des revendications 1 à 8, où le(s) terminal(terminaux) acide(s) C-H du(des) polymère(s) est/sont fonctionnalisé(s) avec un radical C₁-C₁₀-alkyle linéaire ou ramifié ou un radical C₂-C₁₀-alcényle linéaire ou ramifié comprenant au moins une double liaison dont au moins une est terminale, où le radical C₁-C₁₀-alkyle et le radical C₂-C₁₀-alcényle indépendamment l'un de l'autre sont non substitués ou substitués par un ou plusieurs substituant(s) sélectionnés parmi : C₁-C₁₀-alkoxy, C₁-C₁₀-alkylcarbonyle, C₁-C₁₀-alkoxycarbonyle et nitro, de préférence un radical C₂-C₆-alcényle linéaire ou ramifié comprenant une double liaison terminale, où le radical C₂-C₆-alcényle est non substitué ou substitué avec C₁-C₁₀-alkoxycarbonyle, et mieux encore 2-(C₁-C₁₀-alkoxycarbonyl)allyle.

10. Composition pharmaceutique, comprenant une pluralité de nanoparticules selon l'une quelconque des revendications 1 à 9, et un vecteur pharmaceutiquement acceptable.

11. Procédé pour préparer un polymère ayant un degré défini de polymérisation, le procédé comprenant :
(i) la fourniture d'un monomère comprenant une phase liquide sélectionnée parmi : C₁-C₁₀-alkyle cyanoacrylates et C₁-C₆-alkoxy-C₁-C₁₀-alkyle cyanoacrylates dissouts dans un solvant organique hydromiscible aprotique ou un mélange de deux ou plus solvants organiques hydromiscibles aprotiques, où la phase liquide peut de plus comprise un ou plus d'un C₁-C₃-alkanol ;
(ii) l'ajout d'une ou de plus d'une base sélectionnée parmi des hydroxydes de métal et des alkoxydes de métal à la phase liquide, où, au cas où la base est sélectionnée parmi des hydroxydes de métal et la phase liquide prévue dans (i) ne contient aucun C₁-C₃-alkanol, le(s) hydroxyde(s) de métal est/sont ajouté(s) en présence d'un ou de plus d'un C₁-C₃-alkanol ;
(iii) la permission à un équilibre de processus de dépolymérisation et de repolymérisation de s'établir de telle sorte que le polymère ayant un degré de polymérisation défini soit formé ; et
(iv) la désactivation de la réaction de polymérisation.

12. Procédé selon la revendication 11, dans lequel la désactivation dans l'étape (iv) implique le mélangeage de la phase liquide obtenue dans l'étape (iii) contenant le polymère ayant le degré défini de polymérisation avec une solution d'acide aqueuse afin de précipiter le polymère.

13. Procédé selon la revendication 11 ou la revendication 12, dans lequel la phase liquide obtenue dans l'étape (iii) contenant le polymère ayant un degré défini de polymérisation est mise en réaction avec un composé terminateur de la formule générale X-R³, dans lequel X est un groupe partant et R³ est un radical C₁-C₁₀-alkyle linéaire ou ramifié ou un radical C₂-C₁₀-alcényl linéaire ou ramifié comprenant au moins une double liaison dont au moins une est terminale, où le radicale C₁-C₁₀-alkyle et le radical C₂-C₁₀-alcényle indépendamment l'un de l'autre sont non substitués ou substitués par un ou plusieurs substituant(s) sélectionné(s) parmi C₁-C₁₀-alkoxy, C₁-C₁₀-alkylcarbonyle, C₁-C₁₀-alkoxycarbonyle et nitro, avant la désactivation dans étape (iv), dans lequel de préférence X est un halogène et R³ est un radical C₂-C₆-alcényle linéaire ou ramifié comprenant une liaison double terminale, où le radical C₂-C₆-alcényle est non substitué ou substitué avec C₁-C₁₀-alkoxycarbonyle ; mieux encore le composé terminateur est C₁-C₁₀-alkyl 2-bromométhyl acrylate.

14. Procédé pour préparer des nanoparticules, le procédé comprenant :
(a) la fourniture d'une phase liquide hydrophobe comprenant :
(1) un ou plus d'un polymère comprenant de 60 à 330 constituants monomériques sélectionnée parmi : C₁-C₁₀-alkyle cyanoacrylates et C₁-C₆-alkoxy-C₁-C₁₀-alkyle cyanoacrylates, et ayant une dispersité inférieure à 3,0, et
(2) un ou plus d'un composé transporteur sélectionné parmi : des agents pharmaceutiquement actifs, des agents cosmétiquement actifs et des suppléments nutritifs dissouts dans un solvant organique hydromiscible ou un mélange de deux ou plus solvants organiques hydromiscibles ;
(b) la fourniture d'une phase liquide hydrophile, la phase liquide hydrophile comprenant de préférence un, ou plus d'un, agent stabilisateur de nanoparticule sélectionné parmi : des acides biliaires, des sels d'acides biliaires, et des mélanges de ceux-ci, et le(s) agent(s) stabilisateur(s) de nanoparticule étant dissout(s) dans un solvant hydrophile ;
(c) la dispersion fine de la phase liquide hydrophobe dans la phase liquide hydrophile afin de former une émulsion ; et
(d) l'élimination d'au moins une partie du(des) solvant(s) organique(s) à partir de l'émulsion afin d'obtenir une suspension de nanoparticules dans le solvant hydrophile.

15. Nanoparticule, pouvant être obtenue par le procédé selon la revendication 14.
